# EUROPEAN PATENT APPLICATION

(11) **EP 2 607 355 A1**
(43) Date of publication of application: **26.06.2013**
(21) Application number: 11195508.4
(22) Date of filing: 23.12.2011
(51) Int. Cl.: C07D 239/46, C07D 317/44, C07D 487/04, A61K 31/519

(54) **Synthesis of triazolopyrimidine compounds**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to the field of organic synthesis and describes the synthesis of specific intermediates suitable for the preparation of triazolopyrimidine compounds such as ticagrelor.

## Description

The present invention relates to the field of organic synthesis, in particular to the synthesis of specific triazolopyrimidine compounds and intermediates thereof as well as related derivatives.

An important triazolopyrimidine compound is ticagrelor (TCG; Brilinta^{®}; 3-[7-[[(1*R*,2*S*)- 2-(3,4-difluorophenyl)cyclopropyl]amino]-5-(propylthio)-3*H*-1,2,3-triazolo[4,5-d]pyrimidin-3-yl]-5-(2-hydroxyethoxy)-(1*S*,2*S*,3*R*,5*S*)-1,2-cyclopentanediol) having the following structural formula.

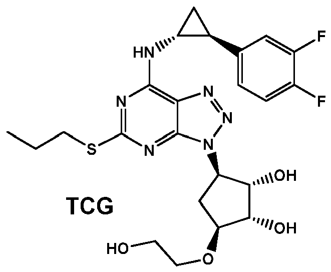

Ticagrelor shows pharmaceutical activity by functioning as a P2Y12 receptor antagonist and thus is indicated for the treatment or prevention of thrombotic events, for example stroke, heart attack, acute coronary syndrome or myocardial infection with ST elevation, other coronary artery diseases and arterial thrombosis as well as other disorders related to platelet aggregation (WO 00/34283).

The synthesis of ticagrelor (**TCG**) is demanding. There are five to six known synthetic variants, which are described in the basic patent application WO 00/34283, an improved one in patent application WO 01/92263, and a further improved one in patent application WO 10/030224 respectively derived from the originator AstraZeneca, while two are published in a "deutero" patent application WO 11/017108 of Auspex Pharmaceuticals. Further, there is one synthetic path published in a scientific journal (Bioorg. Med. Chem. Lett. 2007, 17, 6013-6018). The first synthesis of **TCG** as described in WO 00/34283 is depicted in scheme 1 below.

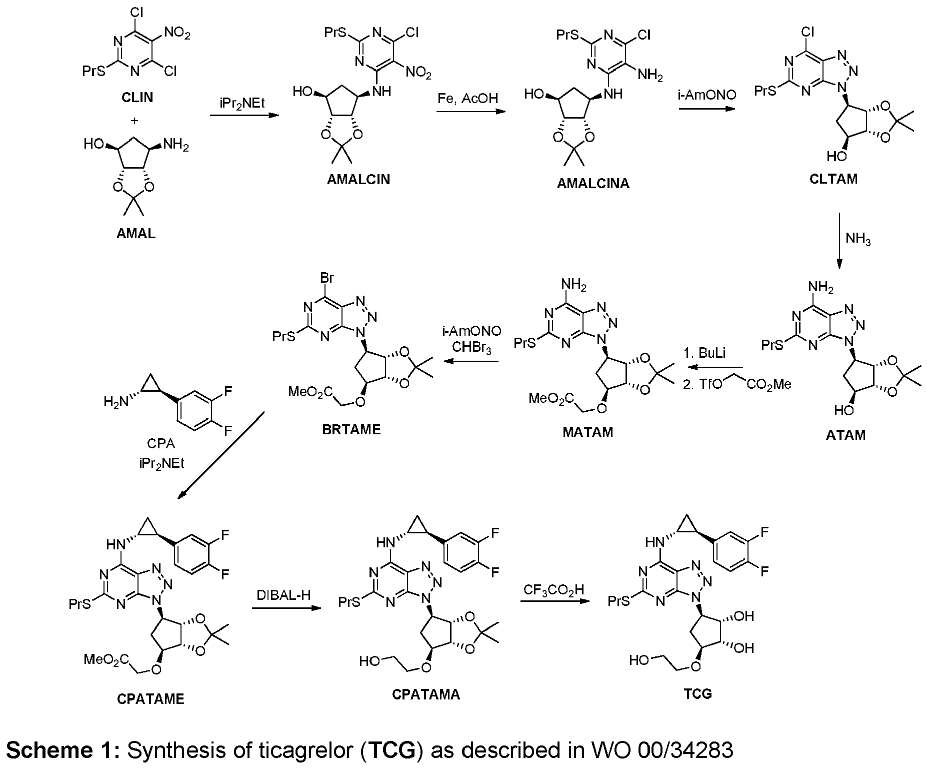

This nine step synthesis of ticagrelor **(TCG)** as described in WO 00/34283 (Scheme 1) starts with a reaction between **CLIN** and **AMAL.** In the presence of diisopropylethylamine (*i*Pr₂Net) **AMALCIN** is formed, which is in then reduced with iron (Fe) in acetic acid to **AMALCINA.** In the next step **CLTAM** is formed using isopentyl nitrite (*i*AmONO). Next, **ATAM** was prepared using ammonia, and side chain was introduced (**MATAM**) using *n*-butyllithium and methyl 2-(((trifluoromethyl)sulfonyl)oxy)acetate, which was previously prepared by reaction between methyl glycolate and triflic anhydride. In next step **BRTAME** is formed using *i*AmONO and CHBr₃, followed by the nucleophilic aromatic substitution of Br with **CPA** in the presence of *i*Pr₂NEt to form **CPATAME.** This is then reduced to **CPATAMA** using DIBAL-H. Deprotection of diol group in the presence of trifluoroacetic acid in the final step leads to **TCG.**

This synthetic path is very long (9 steps, not including reagents preparation) and uses toxic compounds like CHBr₃, triflic anhydride, and methyl 2-(((trifluoromethyl)sulfonyl)oxy)acetate. The introduction of the methoxycarbonylmethyl group (reaction from **ATAM** to **MATAM**) is very difficult due to poor chemoselectivity, as the amino group also reacts with 2-(((trifluoromethyl)sulfonyl)oxy)acetate.

An improved synthesis of ticagrelor (**TCG**) is described in WO 01/92263 (see Scheme 2). In this process the hydroxyethyl side chain is introduced at the beginning of the synthesis by a three step reaction path from **AMAL** to **AMALA,** which is then reacted with **CLINA** (prepared from **CLIDA**) in presence of triethylamine (Et₃N) to form **AMALCINAA.** The triazole ring of **CLTAM** is formed with NaNO₂ in acetic acid, and then Cl is exchanged with **CPA** to form **CPATAMA.** In the final step **TCG** is prepared via deprotection using HCl.

This improved process still has substantial length (7-8 steps). In **AMALA** synthesis the benzyloxycarbonyl protection (*Cbz*) is used, which is then removed in the third step using hydrogenation with Pd/C as a catalyst. Hydrogenation with Pt/C as a catalyst is also used in the reduction of **CLIDA** to **CLINA.**

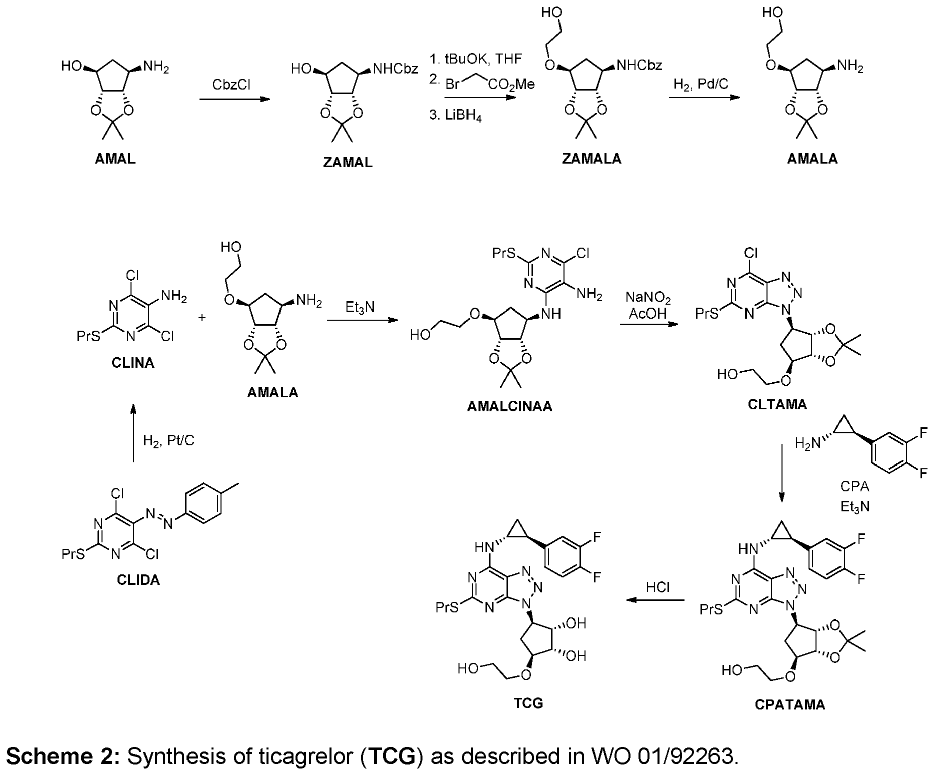

Another improved synthetic path is described in WO 10/030224 (Scheme 3). The key steps in this process are reduction of **CLIN** to **CLINA** or **AMALCINO** to **AMALCINAA** using hydrogen gas and platinum vanadium catalyst. The introduction of the hydroxyethyl side chain to **AMAL** to form **AMALA,** cyclization, substitution of Cl atom of **CLTAMA** with **CPA** and final acidic deprotection are the same as in WO 01/92263.

This further improved process to **TCG** has 8 reaction steps. Like in WO 01/92263, there are used the *Cbz* protecting group and heavy metals as catalysts like Pd, Pt and/or V.

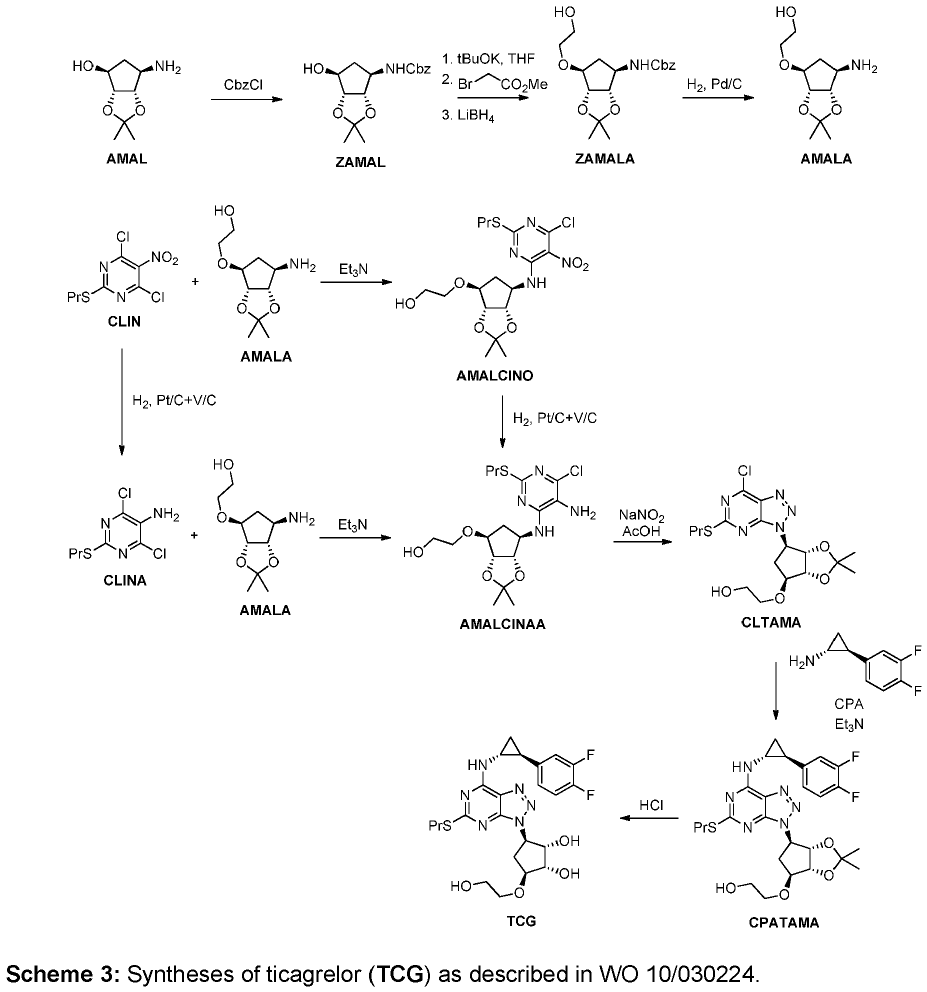

AstraZeneca published a synthetic path (Scheme 4) to ticagrelor (TCG) in Bioorg. Med. Chem. Lett. 2007, 17, 6013-6018. Intermediates in this process are similar to those described in WO 01/92263. There is difference in formation of triazolo ring of **CLTAMA** where iAmONO is used, and difference in deprotection in the last step.

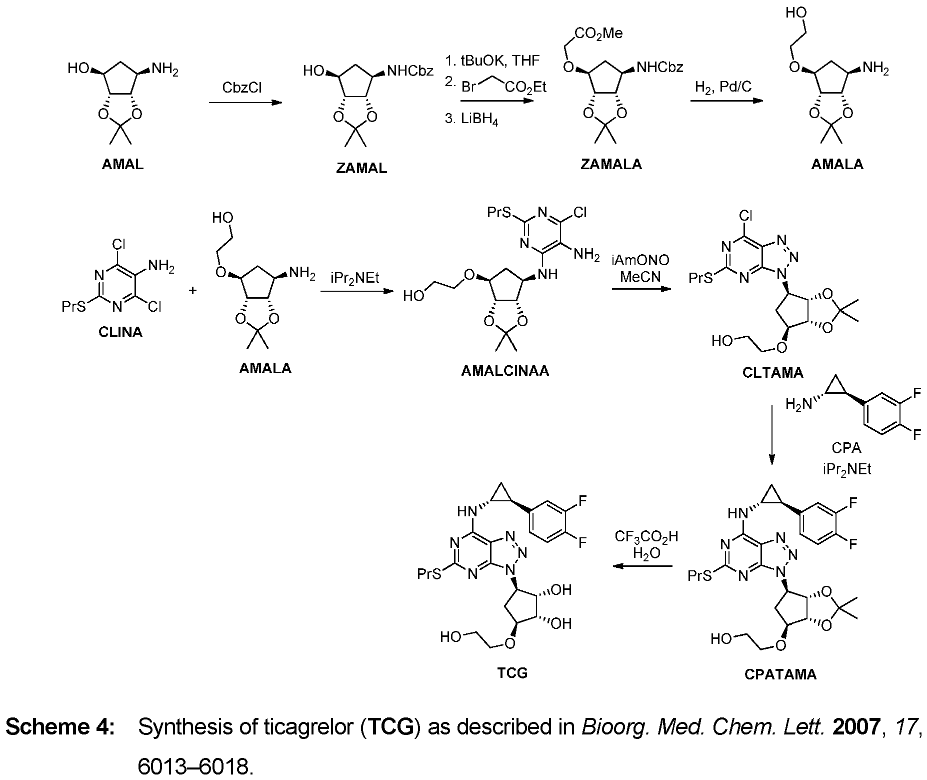

Another synthetic variant (Scheme 5) to ticagrelor (**TCG**) is described in WO 11/017108 by Auspex Pharmaceuticals. In nine step synthesis they prepared **AMALE** through deprotection of **ZAMALE** using hydrogen gas and Pd/C, which was then reduced to **AMALA** with LiAIH₄-**AMALCINO** was prepared without presence of base, further steps are similar to those published in WO 01/92263.

Still another synthetic variant (Scheme 6) to obtain ticagrelor with deuterated hydroxyethyl group (**TCGD**) is also described in WO 11/017108 by Auspex Pharmaceuticals. 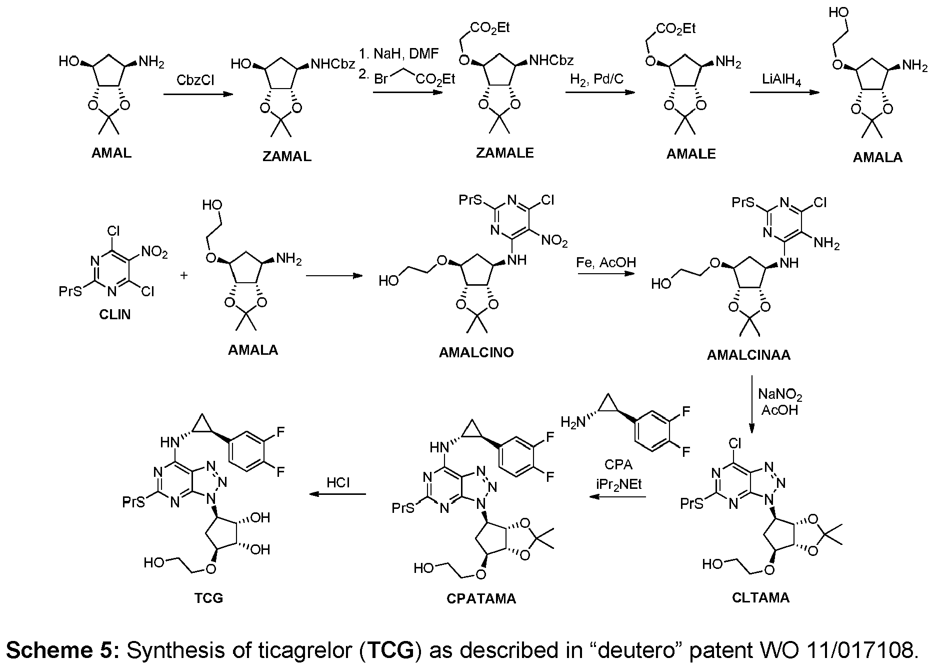 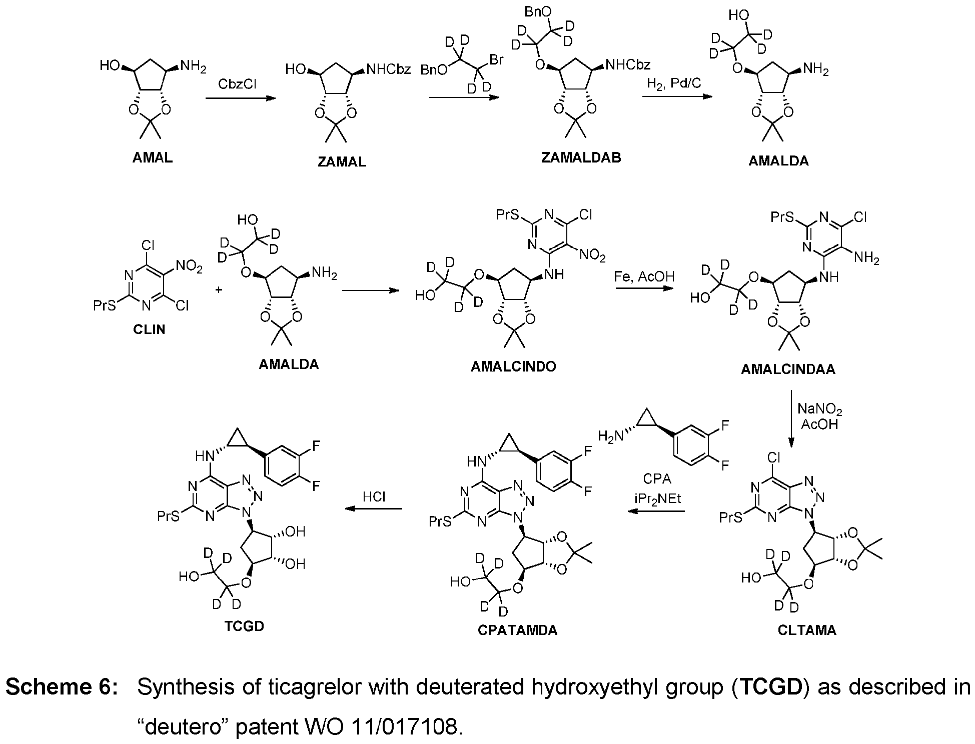 As becomes apparent from the above, a major drawback of the hitherto known synthesis schemes for the preparation of ticagrelor is that the synthesis is long.

### Summary of the Invention

The object of the present invention was to provide an industrially applicable and economically improved process for obtaining ticagrelor.

The present invention provides a process for the preparation of a compound of formula XI (Ticagrelor) or pharmaceutically acceptable salt thereof 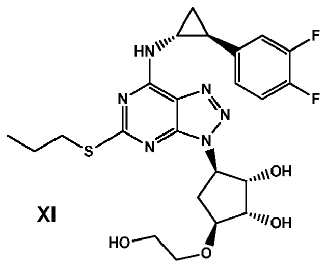 comprising the steps of:
(i) contacting a compound of formula VI or a salt thereof 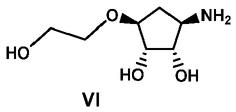 with a compound of formula VII or VII' 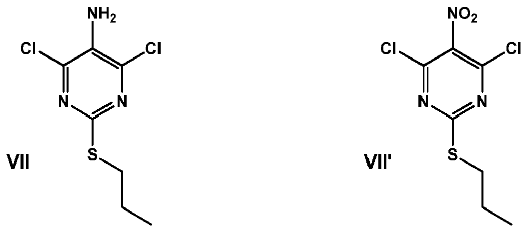 to obtain a compound of formula VIII or VIII', respectively 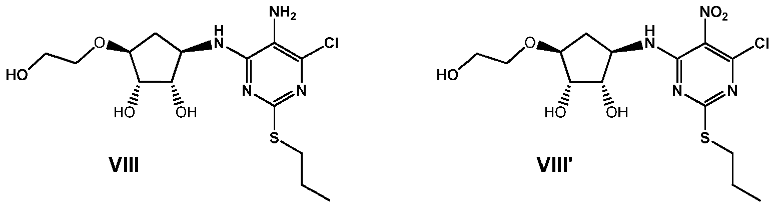
(ii) optionally, if a compound of formula VIII' is obtained, reducing the compound of formula VIII' to a compound of formula VIII,
(iii) converting a compound of formula VIII by nitrosation to a compound of formula IX 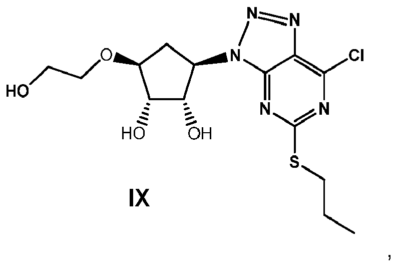 and
(iv) coupling the compound of formula IX with a compound of formula X 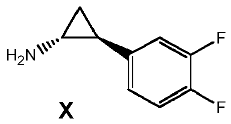
or a salt thereof in a presence of a base to provide a compound of formula XI or a pharmaceutically acceptable salt thereof.

The process defined above allows for preparation or synthesis of ticagrelor with an industrially applicable and economically improved process. Preferred embodiments will be described below. The present invention further provides novel compounds that are highly useful as key intermediates in the preparation or synthesis of ticagrelor.

According to a further aspect, the present invention provides novel and useful process alternatives (i), (ii) and (iii) to prepare a compound of formula VI or a salt thereof, 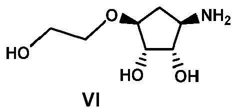 which process alternatives (i), (ii) and (iii) respectively comprises the steps of:
(i) either:
   (0-1) providing a compound of formula III or a salt thereof 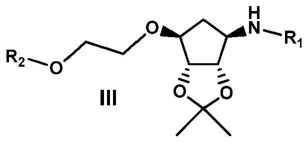 wherein R₁ is benzyl (Bn), *tert*-butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert*-butyl (t-Bu) or fluorenylmethoxycarbonyl (Fmoc), and R₂ is benzyl (Bn), *tert*-butyl (t-Bu), tert-butyldimethylsilyl (TBDMS), methoxymethyl (MOM), acetyl (Ac) or benzoyl (Bz);
   (0-2) directly converting a compound of formula III to a compound of formula VI, and
   (0-3) optionally converting a compound of formula VI to a salt thereof; or:
   (0-1') providing a compound of formula I II or a salt thereof 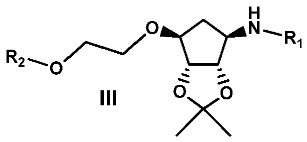 wherein R₁ and R₂ is as defined above,
   (0-2') converting a compound of formula III to a compound of formula IV 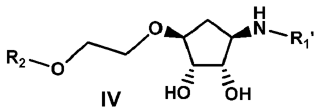 wherein R₁' is hydrogen, Bn, Boc, Cbz, TFA, Tr, TCA, CHO, Ac, Bz, t-Bu or Fmoc, and R₂ is as defined above, and
   (0-3') converting a compound of formula IV to a compound of formula VI,
   (0-4') optionally converting a compound of formula VI to a salt thereof; (iii) or:
   (0-1") providing a compound of formula III or a salt thereof 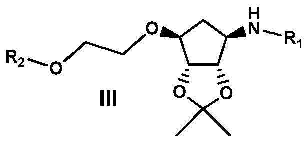 wherein R₁ is benzyl (Bn), *tert*-butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert*-butyl (t-Bu) or fluorenylmethoxycarbonyl (Fmoc), and R₂ is as defined above,
   (0-2") converting a compound of formula III to a compound of formula V 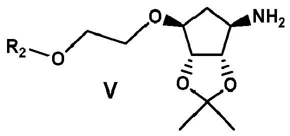
   wherein R₂ is as defined above, and
(0-3") converting a compound of formula V to a compound of formula VI,
(0-4") optionally converting a compound of formula VI to a salt thereof.

For the above alternative embodiments (i), (ii) and (iii), the compound of formula III or a salt thereof may preferably be prepared by comprising the steps of:
providing a compound of formula I 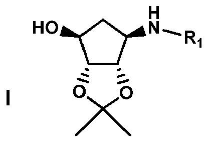 wherein R₁ is Bn, Boc, Cbz, TFA, Tr, TCA, CHO, Ac, Bz, t-Bu or Fmoc, reacting the compound of formula I with a compound of formula II 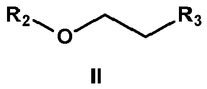
wherein R₂ is Bn, *t*-Bu, TBDMS, MOM, Ac or Bz, and R₃ is TsO (tosylate), MsO (mesylate), Br or Cl
to yield a compound of formula III, and
optionally converting the compound of formula III to a salt thereof.

This further aspect of the invention allows a very short process for introduction of hydroxyethyl side chain to amino carbasugar (2-3 steps), while at the same time using cheap, non-toxic and basic reagents.

Moreover, introduction of hydroxyethyl side chain based on such concept allows to make beneficial use thereof in other synthesis routes to finally obtain obtaining triazolopyrimidine compounds and specifically ticagrelor, as it facilitates introduction (and optionally "pulling through" in subsequent synthetic steps) of a structural moiety "convertible to hydroxyethyl" at any time of the total synthesis desired.

Therefore, another aspect of the present invention resides in that the compound 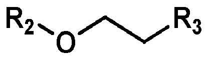 wherein R₂ is benzyl (Bn), tert-butyl (*t*-Bu), *tert*-butyldimethylsilyl (TBDMS), methoxymethyl (MOM), acetyl (Ac) or benzoyl (Bz) and R₃ is TsO, MsO, Br or Cl, is used to introduce "hydroxyethyl convertible" groups into, or to modify, intermediate compounds at desired steps of the synthesis of ticagrelor. Specifically, in such uses and process steps, a structural group denoted as "Z" and defined in the following 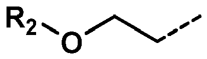 can be used and is regarded as a "group convertible to hydroxyethyl", noting that the hatched bond indicated in the aforementioned formula will be bound to the corresponding oxygen atom of the cyclopentyl ring that is present in each of the intermediate compounds of interest in the synthesis of ticagrelor, corresponding to the following general structural moiety (where the coupling to the above Z="group convertible to hydroxyethyl" is correspondingly shown by a dashed line, while other structural annexes of the respective intermediate compounds of interest are indicated by wavy lines for the ease of illustration):

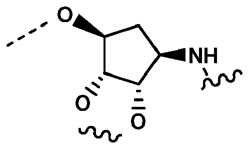

### Description of the Invention and of Preferred Embodiments

Aspects, advantageous features and preferred embodiments of the present invention will be described in further detail below, noting however that such aspects, advantages features as well as embodiments and examples are presented for illustrative purposes only and shall not limit the invention in any way.

Use of intermediate VI as a starting reagent in the synthetic preparation of ticagrelor is a significant feature of the present invention, as it has been found that it allows for a shorter synthesis. This crucial point of using this intermediate distinguishes over intermediate **AMALA** used in the prior art synthesis in the fact that **AMALA** bears an acetonide protecting group, while the hydroxyl groups of the cyclopentane ring of the intermediate VI are not protected. As a consequence, a deprotection step otherwise required in the last step of the preparation of ticagrelor is thus omitted. The omission of said deprotection step, which is conducted in an acidic medium (aq. HCl), does not only result in shortening the synthesis for one reaction step, but also prevents formation of side products, which can be formed if the conventionally used prescribed reaction time and conditions are not followed precisely, for example products of undesired epimerizations.

Further, contrary to the prior art syntheses, the process of the current invention allows the possibility of providing solid crystalline intermediates, which is of significant advantage for enhancing purity of the product of interest. With this preferred embodiment a tedious step of additional purification, which is needed if the final intermediate is not solid (as is the case with known syntheses), can be omitted.

The above advantages are particularly important for preparation of compounds such as ticagrelor. Ticagrelor has 6 stereogenic centers (64 possible isomers), therefore the control of the stereoisomeric impurities during the synthesis is of great significance. If the intermediate entering the final step of the reaction can preferably be isolated in a solid form, the stereoisomeric impurities introduced with the starting material, or formed during the previous steps, can be substantially removed, which offers the possibility of obtaining ticagrelor of improved purity.

A further significant advantage of the present invention resides in the possibility that several steps can, if desired, be performed through one-pot conversions, without the need of isolation or separation of intermediate compounds, which one-pot system therefore constitutes a preferred embodiment of the present invention.

Accordingly, the possibility of reducing the number of required reaction steps and of simplifying reactions respectively strongly contributes to provide an improved industrially applicable and economically beneficial process for obtaining triazolopyrimidine compounds and specifically ticagrelor.

According to a preferred embodiment, the compound of formula VI or a salt thereof is prepared by comprising the steps of
(0-1) providing a compound of formula III or a salt thereof 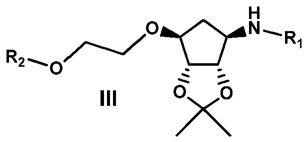 wherein R₁ is benzyl (Bn), *tert*-butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert*-butyl (*t*-Bu) or fluorenylmethoxycarbonyl (Fmoc), and R₂ is benzyl (Bn), *tert*-butyl (t-Bu), *tert*-butyldimethylsilyl (TBDMS), methoxymethyl (MOM), acetyl (Ac) or benzoyl (Bz);
(0-2) directly converting a compound of formula III to a compound of formula VI, and
(0-3) optionally converting a compound of formula VI to a salt thereof.

Alternatively, the compound of formula VI or a salt thereof can be obtained by first carrying out a deprotection reaction of the cyclopentane hydroxyl group and subsequently converting the compound of formula IV to a compound of formula VI, by comprising the steps of:
(0-1') providing a compound of formula III or a salt thereof 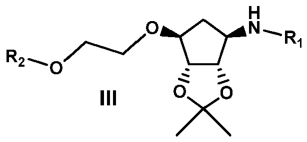 wherein R₁ and R₂ is as defined above,
(0-2') converting a compound of formula III to a compound of formula IV 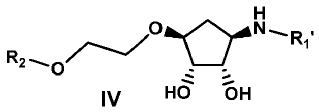 wherein R₁' is hydrogen, Bn, Boc, Cbz, TFA, Tr, TCA, CHO, Ac, Bz, t-Bu or Fmoc, and R₂ is as defined above, and
(0-3') converting a compound of formula IV to a compound of formula VI,
(0-4') optionally converting a compound of formula VI to a salt thereof.

Alternatively, the compound of formula VI or a salt thereof can be obtained by first carrying out a deprotection reaction of the amino group and subsequently converting the compound of formula V to a comopund of formula VI, by comprising the steps of:
(0-1") providing a compound of formula III or a salt thereof 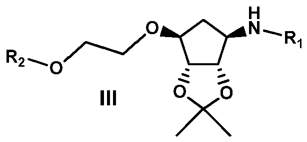 wherein R₁ is benzyl (Bn), *tert*-butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert*-butyl (*t*-Bu) or fluorenylmethoxycarbonyl (Fmoc), and R₂ is as defined above,
(0-2") converting a compound of formula III to a compound of formula V 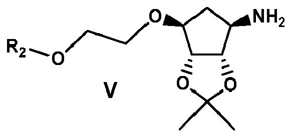 wherein R₂ is as defined above, and
(0-3") converting a compound of formula V to a compound of formula VI,
(0-4") optionally converting a compound of formula VI to a salt thereof.

A summary of the afore-mentioned ways to prepare the compound of formula VI is shown in the following scheme 7 below.

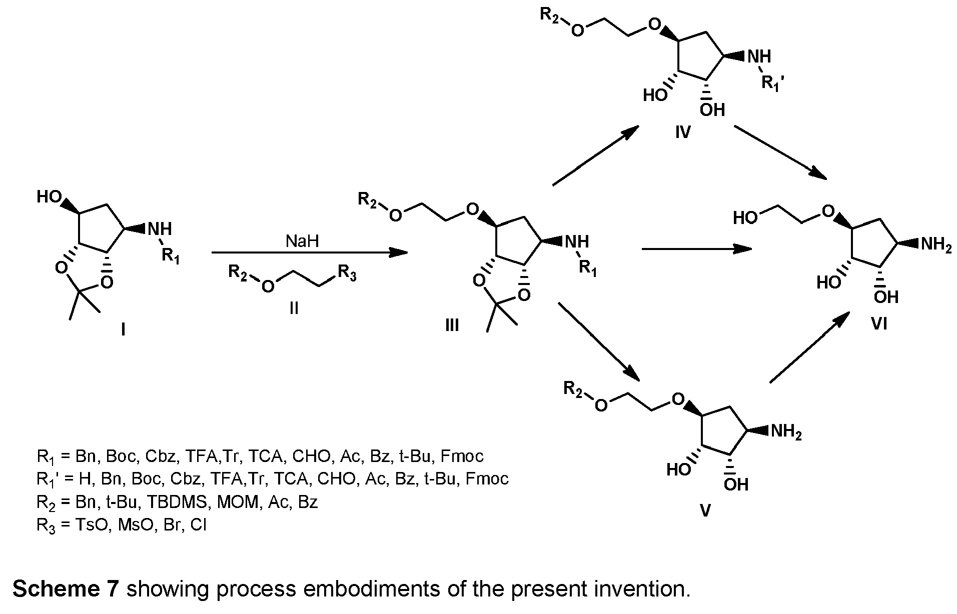

Alternatively, a compound of formula VI can be prepared for example by acid hydrolysis from **AMALA** (Scheme 8), which can be prepared as described in WO 01/92263.

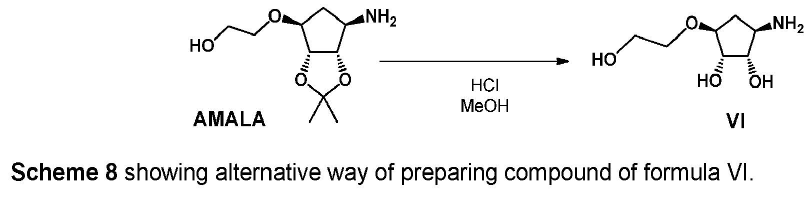

In another embodiment of the present invention presented in scheme 7, the compound of formula III or a salt thereof is prepared by comprising the steps of
(i) providing a compound of formula I 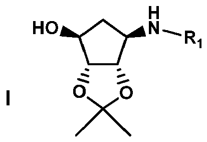 wherein R₁ is Bn, Boc, Cbz, TFA, Tr, TCA, CHO, Ac, Bz, t-Bu or Fmoc,
(ii) reacting the compound of formula I with a compound of formula II 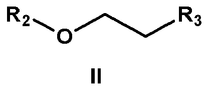 wherein R₂ is Bn, t-BU, TBDMS, MOM, Ac or Bz, and R₃ is tosylate (TsO), mesylate (MsO), Br or Cl to yield a compound of formula III, and
(iii) optionally converting the compound of formula III to a salt thereof.

Such a preferred preparation of compound III is advantageous, when the protection of amino group is introduced during the cyclopentane ring construction. Thus, according to J. Chem. Soc. Perkin Trans. 1, 1994, 613, the compound Ia (R₁=Bn) is a direct product of the synthesis from D-ribose and no subsequent protection is needed.

The substituent R₁ as mentioned above can be any suitable amino protecting group known to a skilled person, for example such a group can be selected from the group consisting of benzyl (Bn), *tert*-butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert-*butyl (*t*-Bu) and fluorenylmethoxycarbonyl (Fmoc).

The substituent R₂ as mentioned above can be any suitable hydroxyl protecting group known to a skilled person, for example such a group can be selected from the group consisting of benzyl (Bn), *tert*-butyl (*t*-Bu), *tert*-butyldimethylsilyl (TBDMS), methoxymethyl (MOM), acetyl (Ac) and benzoyl (Bz).

The substituent R₃ according to the present invention can be any suitable leaving group known to a skilled person, for example sulfonate such as tosylate (TsO) or mesylate (MsO), or halogen such as Br and Cl.

A suitable salt of intermediates III, IV, V and VI is a salt of organic acid, for example an organic achiral acid such as acetic, trifluoroacetic, oxalic, maleic, fumaric or *p*-toluenesulphonic acid, or an organic chiral acid such as L-tartaric acid, dibenzoyl-L-tartaric acid or di-*p*-toluoyl-L-tartaric acid. Preferred salts of intermediates III, IV, V and VI are fumarate, maleate and oxalate. Preparation of a solid salt offers an opportunity for purification of intermediates in order to prepare the compound of formula VI (**OLA**) and consequently ticagrelor of high purity. A further advantage of this preferred possibility of using respective salts resides in that the salts of the intermediately obtained compound of formula VI (**OLA**) are amendable to being used in further subsequent reaction steps according to preferred embodiments and also in an optional one-pot process without intermittent isolation procedures, thereby additionally benefiting from the possibility of further ease and efficiency of final isolation/purification.

As mentioned above, the particularly preferred embodiment of the present invention is a process for preparing a compound of formula XI (ticagrelor, **TCG**), which is presented in scheme 9.

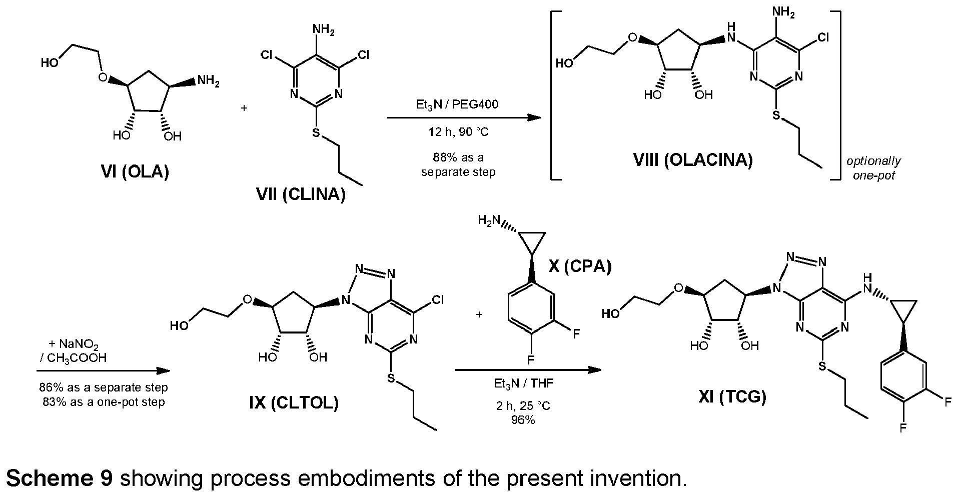

The free base aminoalcohol **OLA** is viscous syrup that has poor solubility in aprotic solvents such as acetone, acetonitrile, tetrahydrofuran, ethyl acetate, dichloromethane and toluene, but is very soluble in lower alcohols and water. It is also relatively well soluble in very polar aprotic solvents such as dimethyl sulfoxide (DMSO), *N,N*-dimethylformamide (DMF) and N-methylpyrrolidin-2-one (NMP), or in a solvent such as polyethyleneglycol (PEG). In particular, a certain amount of polyethyleneglycol to solubilize **OLA, CLINA** and triethylamine was found suitable as a solvent system for the initial *N*-arylation reaction. Alternatively, alcohols such as 2-propanol, butanols or pentanols can be used. Polyethyleneglycol is a non-toxic, inexpensive and non-volatile solvent that can be easily removed by an aqueous wash. This solvent also allows for the use of bases other than triethylamine, such as sodium or potassium carbonates or bicarbonates. Generally, 1 to 1.5 equivalents of base is used. If a salt of **OLA** is chosen, then 2 to 2.5 equivalents of base is used. The reaction can also be efficiently performed in the absence of any added solvent by using triethanolamine as a base. Triethanolamine is a tertiary amine that is miscible with both other reactants, **OLA** and **CLINA.** The formation of **OLACINA** requires temperatures of 60 to 100°C to achieve a satisfactory reaction time. All these bases, triethylamine, triethanolamine and alkali (bi)carbonates, with their pertaining reaction modifications, are suitable for the one-pot integration of the subsequent nitrosation of the formed **OLACINA.** In this respect, it is not necessary to isolate **OLACINA,** because yields are improved when applying the one-pot protocols for the synthesis of **CLTOL** and its impurity profile is not substantially affected. The nitrosation is performed by diluting the reaction mixture with acetic acid and slowly adding sodium nitrite while cooling to about 20°C. The product **CLTOL** is isolated by dilution with water and extraction. If required, it can be purified by either precipitation from its solutions using an antisolvent such as n-heptane, or recrystallized from solvents such as for example methyl tert-butyl ether or similar. The reaction of **CLTOL** with the cyclopropylamine **CPA** is rapid already at ambient temperature (25°C) and gives ticagrelor in a nearly quantitative yield. Solvent used for this reaction step is for example tetrahydrofuran to which triethylamine as a scavenger for the formed HCl can be added. Instead of **CPA** base, salts of **CPA** can also be directly used in this reaction step. For example, **CPA** mandelate in acetonitrile, with sodium carbonate as a base, gives ticagrelor in a 93% yield. Overall, the total yield of ticagrelor starting from **OLA** is about 80%.

Alternatively, the N-arylation of **OLA** can also be performed with the nitropyrimidine **CLIN** to give crystalline intermediate **OLACIN** in a 70% yield (Scheme 10). While the selectivity for the monosubstitution of a single chlorine in the nucleophilic aromatic substitution on **CLIN** is slightly reduced, the advantage of using the nitropyrimidine **CLIN** is in its high reactivity allowing the reaction to take place in matter of minutes even at 0 °C. With reduction of the nitro group, the common intermediate **OLACINA** is obtained in a pure form without resorting to chromatography. The reduction of the nitro group can be performed by the hydrogenation of **OLACIN** in acetic acid. The so obtained solution of **OLACINA** in acetic acid is easily nitrosated by the use of sodium nitrite to give the pyrimidinotriazole intermediate **CLTOL.** In this manner, **CLTOL** is obtained in a 71% overall yield from **OLACIN** by what is essentially a one-pot process. Ticagrelor is then prepared from **CLTOL** and **CPA** using the same method as in the original synthetic variant (48% total yield from **OLA**).

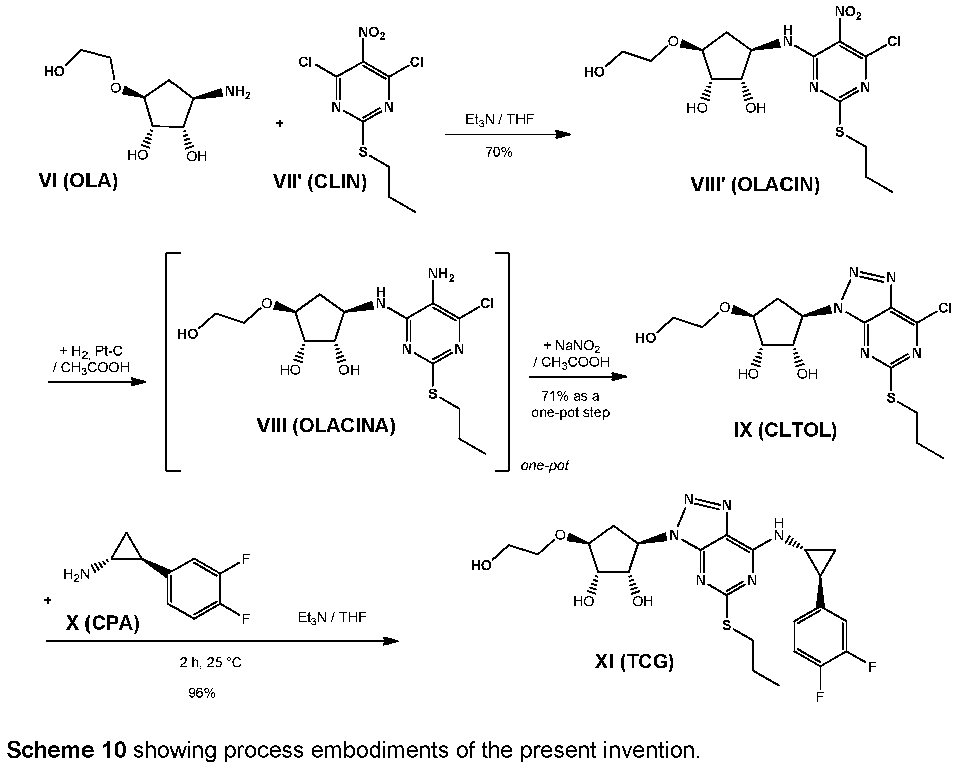

As set forth above, it is possible and corresponds to a particularly preferred embodiment of the present invention that the intermediate VIII is not isolated. Thus, while of course separation or isolation of the intermediate compound of formulae VIII can be carried out to obtain such compound as useful intermediate compound, this can be beneficially dispensed with if desired. This preferred embodiment is not only economically beneficial by the feature that one-pot synthesis is made possible; it is especially advantageous due to the generally amorphous nature of the intermediate compounds, which would make the purification difficult using non-chromatographic means, while the use of chromatographic means would again render the whole process less economically acceptable.

The ticagrelor compound prepared according to the invention may be used or administered on its own, preferably it is administered as a pharmaceutical composition comprising ticagrelor and a pharmaceutically acceptable excipient and/or carrier. Further, the ticagrelor compound prepared according to the invention may be combined with other drugs, especially drugs having activity against platelet aggregation or thrombolytic events.

In a further aspect of the present invention, a pharmaceutical composition comprising the compound of formula XI (ticagrelor, TCG) or a salt thereof is prepared by comprising the steps of preparing the compound of formula VIII or a salt thereof as described above, and mixing the compound of formula XI or a salt thereof with a pharmaceutically acceptable carrier and/or excipient. The administration form can be suitably chosen, e.g. a form suitable for oral, parenteral, rectal administration and/or administration by inhalation, and the dosage form may be solid, liquid, or powdery. Therefore, the pharmaceutical composition comprising ticagrelor compound prepared according to the invention may suitably be in the form of tablets, pills, capsules, syrups, powders or granules for oral administration; or as sterile parenteral or subcutaneous solutions, suspensions for parenteral administration; or as suppositories for rectal administration.

Suitable excipients and/or carriers include, without being limited to, diluents, binders, disintegrants, lubricants, etc. For example, the compound or a finely divided form thereof, or particles comprising the compound, are mixed with a carrier or binder substance, e.g. a mono-, di- or polysaccharide such as sugars and starch, a sugar alcohol or another polyol. For example, lactose, saccharose, sorbitol, mannitol, starch, cellulose derivatives, a binder such as polyvinylpyrrolidone, and a lubricant such as magnesium stearate, calcium stearate, polyethylene glycol, waxes, paraffin, and the like are mixed, and then compressed into tablets. The compound or a finely divided form thereof or particles containing the same may be coated by another substance. The powder mixture or particles containing the compound may also be dispensed into capsules.

The pharmaceutical composition comprising ticagrelor prepared according to the invention in a desired dose is generally suitable to treat a disease or condition of a patient in need thereof, specifically to display a desired activity against platelet aggregation, or in the treatment or prophylaxis of thrombolytic events.

Further aspects of the present invention reside in the provision of valuable intermediate compounds III to V and salts thereof, and intermediate compounds VIII, VIII' and IX, all useful in the synthesis of a compound of formula XI (ticagrelor, TCG): 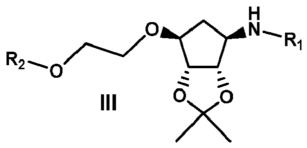 wherein R₁ is benzyl (Bn), *tert*-butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert*-butyl (t-Bu) or fluorenylmethoxycarbonyl (Fmoc), and R₂ is benzyl (Bn), *tert*-butyl (*t*-Bu), *tert-*butyldimethylsilyl (TBDMS), methoxymethyl (MOM), acetyl (Ac) or benzoyl (Bz); 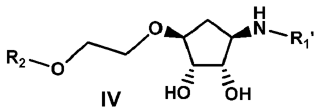 wherein R₁' is hydrogen, Bn, Boc, Cbz, TFA, Tr, TCA, CHO, Ac, Bz, t-Bu or Fmoc, and R₂ is benzyl (Bn), *tert*-butyl (t-Bu), *tert*-butyldimethylsilyl (TBDMS), methoxymethyl (MOM), acetyl (Ac) or benzoyl (Bz); 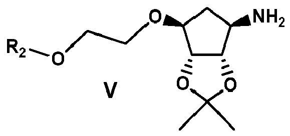 wherein R₂ is benzyl (Bn), *tert*-butyl (*t*-Bu), *tert*-butyldimethylsilyl (TBDMS), methoxymethyl (MOM), acetyl (Ac) or benzoyl (Bz);

A suitable salt of intermediates III, IV and V is a salt of organic acid, for example an organic achiral acid such as acetic, trifluoroacetic, oxalic, maleic, fumaric or *p*-toluenesulphonic acid, or an organic chiral acid such as L-tartaric acid, dibenzoyl-L-tartaric acid or di-p-toluoyl-L-tartaric acid. Preferred salts of intermediates III, IV and V are fumarate, maleate and oxalate.

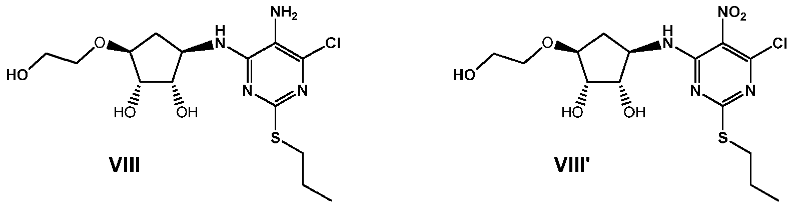

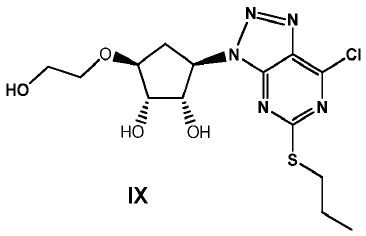

Particular examples of such useful intermediate compounds are listed by their respective formulas below:

| **Formula** | **Chemical name** |
|---|---|
| 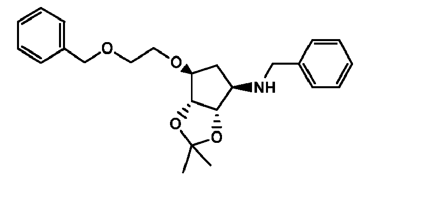 | (3a*S*,4*R*,6*S*,6a*R*)-*N*-Benzyl-6-(2-(benzyloxy)ethoxy)-2,2-dimethyltetrahydro-3aH-cyclopenta[*d*][1,3]dioxol-4-amine |
| 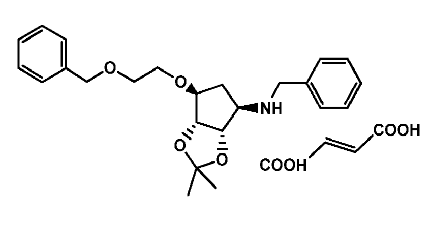 | (3a*S*,4*R*,6*S*,6a*R*)-*N*-Benzyl-6-(2-(benzyloxy)ethoxy)-2,2-dimethyltetrahydro-3a*H*-cyclopenta[*d*][1,3]dioxol-4-amine fumarate |
| 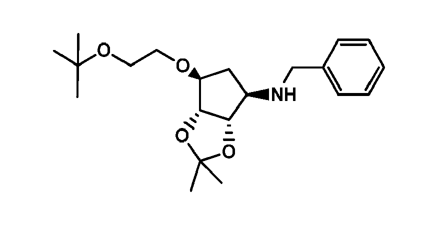 | (3a*S*,4*R*,6*S*,6a*R*)-*N*-Benzyl-6-(2-*tert*-butoxy)ethoxy)-2,2-dimethyltetrahydro-3a*H*-cyclopenta[*d*][1,3]dioxol-4-amine |
| 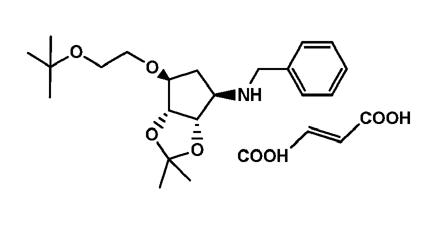 | 3aS,4*R*,6*S*,6a*R*)-*N*-Benzyl-6-(2-*tert*-butoxy)ethoxy)-2,2-dimethyltetrahydro-3aH-cyclopenta[*d*][1,3]dioxol-4-amine fumarate |
| 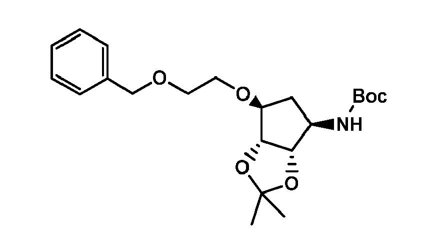 | *tert*-Butyl ((3a*S*,4*R*,6*S*,6a*R*)-6-(2-(benzyloxy)ethoxy)-2,2-dimethyltetrahydro-3a*H*-cyclopenta[*d*][1,3]dioxol-4-yl)carbamate |
| 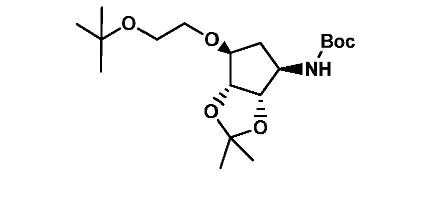 | *tert*-Butyl ((3a*S*,4*R*,6*S*,6a*R*)-6-(2-(*tert*-butoxy)ethoxy)-2,2-dimethyltetrahydro-3a*H*-cyclopenta[*d*][1,3]dioxol-4-yl)carbamate |
| 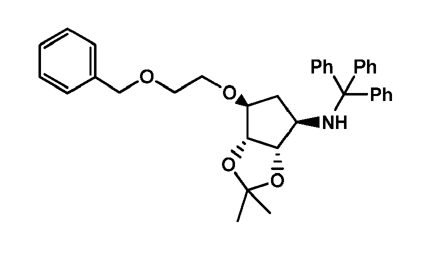 | (3a*S*,4*R*,6*S*,6a*R*)-6-(2-(benzyloxy)ethoxy)-2,2-dimethyl-*N*-trityltetrahydro-3a*H*-cyclopenta[*d*][1,3]dioxol-4-amine |
| 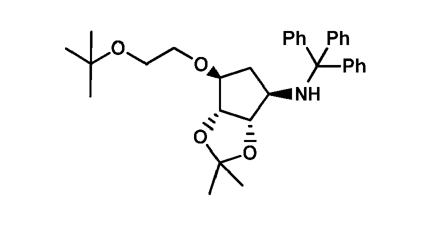 | (3a*S*,4*R*,6*S*,6a*R*)-6-(2-(*tert*-butoxy)ethoxy)-2,2-dimethyl-*N*-trityltetrahydro-3a*H*-cyclopenta[*d*][1,3]dioxol-4-amine |
| 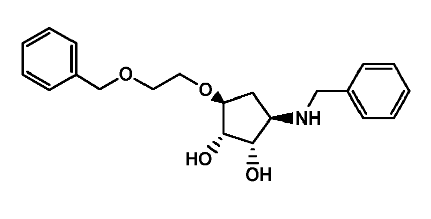 | (1*S*,2*S*,3*R*,5*S*)-3-(Benzylamino)-5-(2-(benzyloxy)ethoxy)cyclopentane-1,2-diol |
| 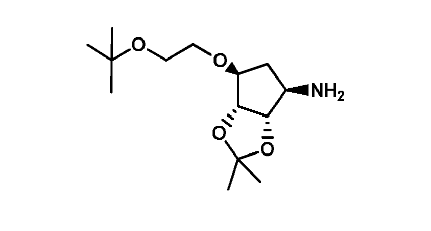 | (3a*S*,4*R*,6*S*,6a*R*)-6-(2-(*tert*-butoxy)ethoxy)-2,2-dimethyltetrahydro-3a*H*-cyclopenta[*d*][1,3]dioxol-4-amine |
| 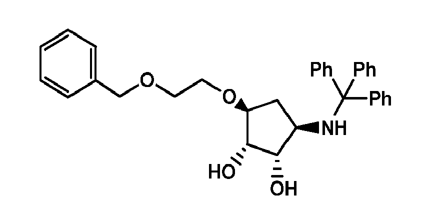 | (1*S*,2*S*,3*S*,5*R*)-3-(2-(benzyloxy)ethoxy)-5-(tritylamino)cyclopentane-1,2-diol |
| 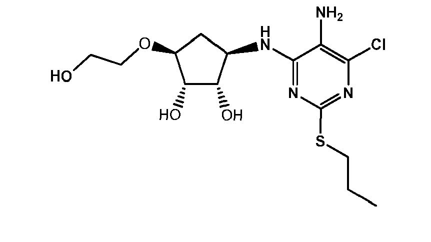 | (1*S*,2*S*,3*R*,5*S*)-3-((5-amino-6-chloro-2-(propylthio)pyrimidin-4-yl)- amino)-5-(2-hydroxyethoxy)cyclopentane-1,2-diol |
| 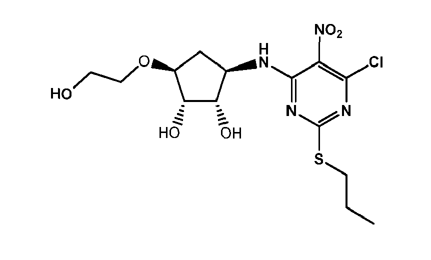 | (1*S*,2*S*,3*R*,5*S*)-3-((6-chloro-5-nitro-2-(propylthio)pyrimidin-4-yl)amino)-5-(2-hydroxyethoxy)cyclopentane-1,2-diol |
| 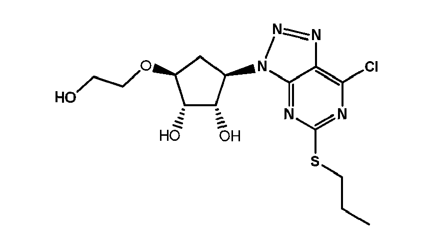 | (1*S*,2*S*,3*R*,5*S*)-3-(7-chloro-5-(propylthio)-3*H*-[1,2,3]triazolo[4,5-*d*]pyrimidin-3-yl)-5-(2-hydroxyethoxy)cyclopentane-1,2-diol |

In the following the present invention will be described in further detail by illustrative, nonlimiting examples.

### Examples

### Example 1: Preparation of (3aS,4R,6S,6aR)-N-Benzyl-6-(2-(benzyloxy)ethoxy)-2,2-dimethyl-tetrahydro-3aH-cyclopenta[d][1,3]dioxol-4-amine (IIIa)

### a)

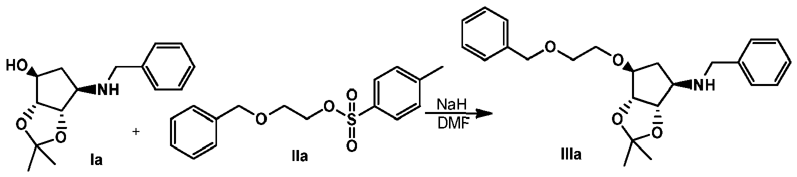

A solution of (3aR,4S,6R,6aS)-6-(benzylamino)-2,2-dimethyltetrahydro-3aH-cyclopenta-[d][1,3]-dioxol-4-ol (1.0 g, 3.8 mmol) in dry DMF (10 mL) under nitrogen atmosphere was cooled at 0 °C followed by addition of NaH (60 %, 182 mg, 4.6 mmol). After stirring for 30 min at 0 °C, 2-(benzyloxy)ethyl 4-methylbenzenesulfonate (1.2 g, 3.8 mmol) was added and the reaction mixture was allowed to warm at room temperature. After stirring for 4 hours, the reaction mixture was quenched with water (10 mL). To the mixture was extracted 3 x 10 mL of n-hexane. The combined organic phases were dried over MgSO₄, filtered and evaporated to the dryness.

### b)

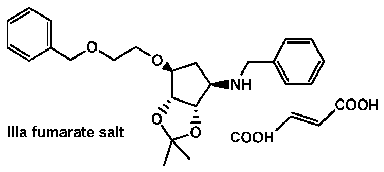

Obtained IIIa was isolated from the reaction mixture by salt formation with fumaric acid. The solution of reaction mixture of IIIa (contained about 80 % of IIIa) in 2-butanone was warmed to 50 °C. 1 eq of fumaric acid (calculated to amount of IIIa) was added and reaction mixture was stirred at 50°C until fumaric acid dissolution. The reaction mixture was allowed to cool at room temperature followed by addition of n-hexane. After overnight stirring at room temperature, the precipitate white salt of IIIa was sucked off, washed with n-hexane and dried under reduce pressure at 40°C.

### c)

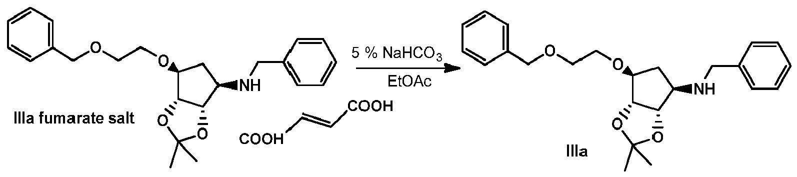

IIIa fumarate salt was suspended in EtOAc and 5% aqueous solution of NaHCO₃ was added to the suspension. The mixture was stirred vigorously at room temperature for an hour. The two clear phases were separated and organic phase was washed with water, dried over MgSO₄ and evaporated to the dryness to provide pure IIIa.
¹H NMR (CDCl₃) δ = 1.30 (s, 3H), 1.40 (s, 3H), 1.90 (d, 1 H), 2.15 (m, 1 H), 3.17 (m, 1H), 3.60 (m, 2H), 3.68 (m, 2H), 3.80-3.90 (m, 3H), 4.51 (s, 2H), 4.63 (m, 2H), 7.23-7.36 (m, 10H) ppm. ¹³C NMR (CDCl₃) δ = 24.0, 26.4, 33.9, 51.6, 63.0, 68.5, 69.2, 73.1, 83.9, 84.6, 84.8 110.4, 126.7, 127.5, 127.53, 128.0, 128.2, 128.3, 138.1, 140.3 ppm.

### Example 2: Preparation of (3aS,4R,6S,6aR)-N-Benzyl-6-(2-tert-butoxy)ethoxy)-2,2-dimethyl-tetrahydro-3aH-cyclopenta[d][1,3]dioxol-4-amine (IIIb)

### a)

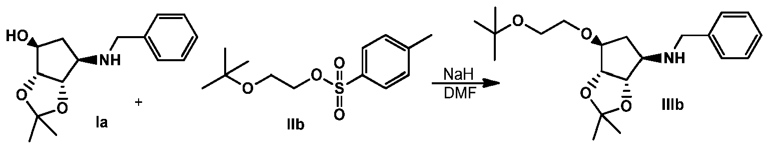

A solution of (3aR,4S,6R,6aS)-6-(benzylamino)-2,2-dimethyltetrahydro-3a*H*-cyclopenta-[*d*][1,3]-dioxol-4-ol (10 g, 38 mmol) in dry DMF (100 mL) under nitrogen atmosphere was cooled at 0 °C followed by addition of NaH (60 %, 1.8 g, 46 mmol). After stirring for 30 min at 0 °C, 2-(*tert-*butoxy)ethyl 4-methylbenzenesulfonate (10.3 g, 38 mmol) was added and the reaction mixture was allowed to warm at room temperature. After stirring for 4 hours, the reaction mixture was quenched with water (100 mL). The mixture was extracted 3 x 10 mL of n-hexane. The combined organic phases were dried over MgSO₄, filtered and evaporated to the dryness.

### b)

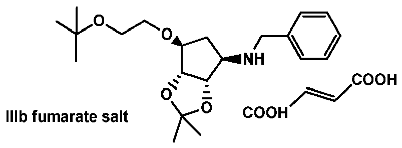

Obtained IIIb was isolated from the reaction mixture by salt formation with fumaric acid. The solution of reaction mixture of IIIb (contained about 60 % of IIa) in 2-butanone was warmed to 50 °C. 1 eq of fumaric acid (calculated to amount of IIIb) was added and reaction mixture was stirred at 50 °C until fumaric acid dissolution. The reaction mixture was allowed to cooled at room temperature followed by addition of n-hexane. After overnight stirring at room temperature, the precipitate white salt of IIIb was sucked off, washed with n-hexane and dried under reduce pressure at 40 °C.

### c)

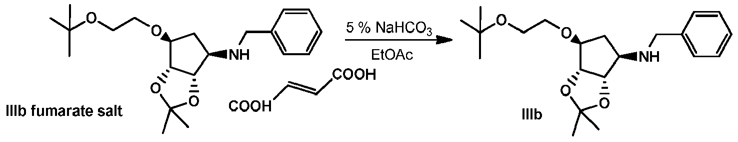

IIb fumarate salt was suspended in EtOAc and 5% aqueous solution of NaHCO3 was added to the suspension. The mixture was stirred vigorously at room temperature for an hour. The two clear phases were separated and organic phase was washed with water, dried over MgSO4 and evaporated to the dryness to provide pure IIIb.
¹H NMR (CDCl₃) δ = 1.14 (s, 9H), 1.30 (s, 3H), 1.40 (s, 3H), 1.88 (d, 1H), 2.10 (m, 1H), 3.14 (m, 1 H), 3.45 (m, 2H), 3.59 (m, 2H), 3.80-3.90 (m, 3H), 4.62 (m, 2H), 7.22-7.35 (m, 5H) ppm. ¹³C NMR (CDCl₃) δ = 24.0, 26.4, 27.4, 33.8, 51.7, 60.1, 63.1, 69.1, 72.9, 83.9, 84.6, 84.8 110.2, 126.7, 128.1, 128.2, 140.3 ppm.

### Example 3: Preparation of tert-Butyl ((3aS,4R,6S,6aR)-6-(2-(benzyloxy)ethoxy)-2,2-dimethyl-tetrahydro-3aH-cyclopenta[d][1,3]dioxol-4-yl)carbamate (IIIc)

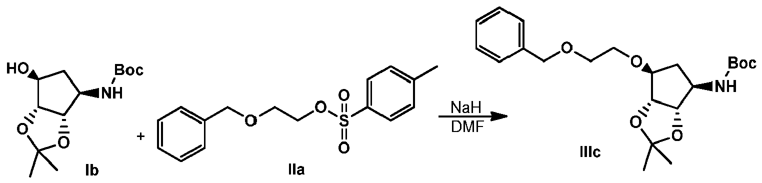

A solution of *tert-*butyl ((3a*S*,4*R*,6*S*,6a*R*)-6-hydroxy-2,2-dimethyltetrahydro-3a*H*-cyclopenta[*d*]-[1,3]dioxol-4-yl)carbamate (3.0 g, 10.8 mmol) in dry DMF (30 mL) under nitrogen atmosphere was cooled at 0 °C followed by addition of NaH (60 %, 530 mg, 13.0 mmol). After stirring for 30 min at 0 °C, 2-(benzyloxy)ethyl 4-methylbenzenesulfonate (3.3 g, 10.8 mmol) was added and the reaction mixture was allowed to warm at room temperature. After stirring for 4 hours, the reaction mixture was quenched with water (30 mL). To the mixture was extracted 3 x 30 mL of n-hexane. The combined organic phases were dried over MgSO₄, filtered and evaporated to the dryness. The pure IIIc was isolated from reaction mixture by chromatography on silica gel column (mobile phase: n-hexane/EtOAc).
¹H NMR (CDCl₃) δ = 1.30 (s, 3H), 1.40 (s, 12H), 1.82 (d, 1H), 2.14 (m, 1H), 3.53-3.67 (m, 4H), 3.87 (d, 1H), 4.11 (m, 1H), 4.49-4.58 (m, 3H), 4.65 (m, 1H), 5.73 (d, 1H), 7.27-7.36 (m, 5H) ppm.
¹³C NMR (CDCl₃) δ = 23.8, 26.2, 28.4, 32.3, 56.4, 68.0, 68.8, 73.2, 79.0, 83.5, 84.9, 86.0, 110.1, 127.7, 128.4, 138.0, 155.2 ppm.

### Example 4: Preparation of tert-Butyl ((3aS,4R,6S,6aR)-6-(2-(tert-butoxy)ethoxy)-2,2-dimethyltetrahydro-3aH-cyclopenta[d][1,3]dioxol-4-yl)carbamate (IIId)

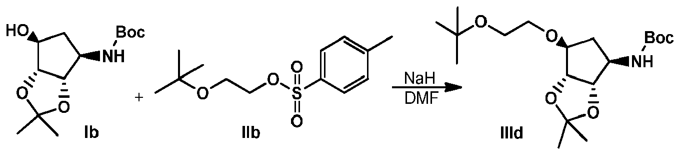

A solution of tert-butyl ((3aS,4R,6S,6aR)-6-hydroxy-2,2-dimethyltetrahydro-3aH-cyclopenta-[d][1 ,3]dioxol-4-yl)carbamate (7.0 g, 25.6 mmol) in dry DMF (70 mL) under nitrogen atmosphere was cooled at 0 °C followed by addition of NaH (60 %, 1.3 g, 30.7 mmol). After stirring for 30 min at 0 °C, 2-(tert-butoxy)ethyl 4-methylbenzenesulfonate (7.0 g, 25.6 mmol) was added and the reaction mixture was allowed to warm at room temperature. After stirring for 4 hours, the reaction mixture was quenched with water (70 mL). To the mixture was extracted 3 x 100 mL of n-hexane. The combined organic phases were dried over MgSO₄, filtered and evaporated to the dryness. The pure IIId was isolated from reaction mixture by chromatography on silica gel column (mobile phase: n-hexane/EtOAc).
¹H NMR (CDCl₃) δ = 1.21 (s, 9H), 1.26 (s, 3H), 1.40 (s, 3H), 1.43 (s, 9H), 1.78 (d, 1H), 2.15 (m, 1H), 3.47-3.57 (m, 3H), 3.65 (m, 1H), 3.83 (m, 1H), 4.10 (m, 1H), 4.50 (m, 2H), 4.60 (m, 1H), 5.50 (d, 1H) ppm.
¹³C NMR (CDCl₃) δ = 23.8, 26.2, 27.5, 32.7, 56.5, 60.9, 69.2, 73.0, 83.3, 85.1, 86.0, 110.0, 128.0, 129.7, 155.2 ppm.

### Example 5: (3aS,4R,6S,6aR)-6-(2-(benzyloxy)ethoxy)-2,2-dimethyl-N-trityltetrahydro-3aH-cyclopenta[d][1,3]dioxol-4-amine (IIIe)

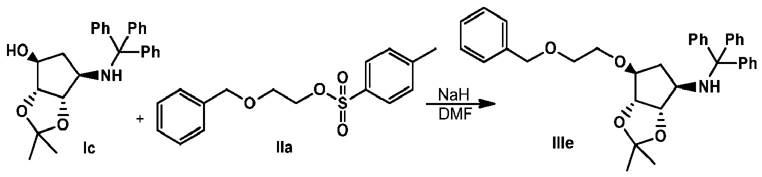

A solution of (10 g, 24.0 mmol) (3aR,4S,6R,6aS)-2,2-dimethyl-6-(tritylamino)tetrahydro-3aH-cyclopenta[d][1,3]dioxol-4-ol in dry DMF (100 mL) under nitrogen atmosphere was cooled at 0 °C followed by addition of NaH (60 %, 1.2 g, 29.0 mmol). After stirring for 30 min at 0 °C, 2-(benzyloxy)ethyl 4-methylbenzenesulfonate (7.4 g, 24.0 mmol) was added and the reaction mixture was allowed to warm at room temperature. After stirring for 4 hours, the reaction mixture was quenched with water (100 mL). To the mixture was extracted 3 x 100 mL of EtOAc. The combined organic phases were dried over MgSO₄, filtered and evaporated to the dryness. The pure IIIe was isolated from reaction mixture by chromatography on silica gel column (mobile phase: n-hexane/EtOAc).
¹H NMR (CDCl₃) δ = 0.57 (d, 1 H), 1.20 (s, 3H), 1.30 (s, 3H), 1.45 (m, 1H), 2.50 (s, 1H), 3.15 (s, 1 H), 3.41 (m, 4H), 3.57 (m, 1 H), 4.55 (s, 2H), 7.19 (m, 3H), 7.26-7.39 (m, 7H), 7.56 (d, 6H) ppm. ¹³C NMR (CDCl₃) δ = 23.8, 26.2, 27.3, 32.5, 59.3, 61.0, 68.8, 71.5, 72.8, 83.7, 85.2, 87.6, 109.4, 126.1, 127.2, 127.8, 128.3, 128.6, 128.7, 146.9 ppm.

### Example 6: Preparation of (3aS,4R,6S,6aR)-6-(2-(tert-butoxy)ethoxy)-2,2-dimethyl-N-trityl-tetrahydro-3aH-cyclopenta[d][1,3]dioxol-4-amine (IIIf)

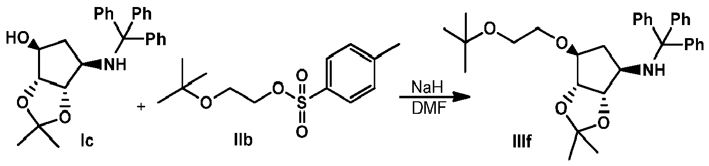

A solution of (3aR,4S,6R,6aS)-2,2-dimethyl-6-(tritylamino)tetrahydro-3aH-cyclopenta[d]-[1,3]dioxol-4-ol (10.0 g, 24.0 mmol) in dry DMF (100 mL) under nitrogen atmosphere was cooled at 0 °C followed by addition of NaH (60 %, 1.2 g, 29.0 mmol). After stirring for 30 min at 0 °C, 2-(tert-butoxy)ethyl 4-methylbenzenesulfonate (6.5 g, 24.0 mmol) was added and the reaction mixture was allowed to warm at room temperature. After stirring for 4 hours, the reaction mixture was quenched with water (100 mL). To the mixture was extracted 3 x 100 mL of EtOAc. The combined organic phases were dried over MgSO4, filtered and evaporated to the dryness. The pure IIIf was isolated from reaction mixture by chromatography on silica gel column (mobile phase: n-hexane/EtOAc).
1 H NMR (CDCl₃) δ = 0.57 (d, 1 H), 1.20 (s, 9H), 1.30 (s, 3H), 1.35 (s, 3H), 1.45 (m, 1 H), 2.50 (s, 1 H), 3.15 (s, 1 H), 3.41 (m, 4H), 3.57 (m, 1 H), 4.55 (s, 2H), 7.19 (m, 3H), 7.26-7.39 (m, 7H), 7.56 (d, 6H) ppm.
13C NMR (CDCl₃) δ = 23.8, 26.2, 27.3, 32.5, 59.3, 61.0, 68.8, 71.5, 72.8, 83.7, 85.2, 87.6, 109.4, 126.1, 127.2, 127.8, 128.3, 128.6, 128.7, 146.9 ppm.

### Example 7: Preparation of (1S,2S,3R,5S)-3-(Benzylamino)-5-(2-(benzyloxy)ethoxy)cyclopentane-1,2-diol (IVa)

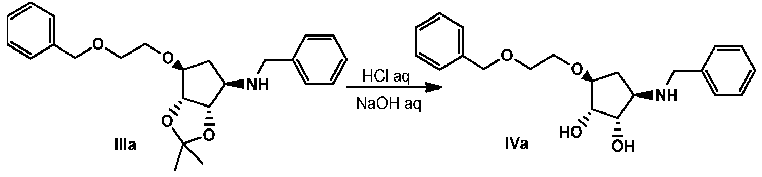

IIIa (2.0 g, 5 mmol) was treated with 1M HCl (20 mL) at room temperature for 7 hours followed by addition of 1M NaOH (25 mL). The reaction mixture was extracted with 30 mL of EtOAc. Organic phase was washed with water, dried over MgSO4 and evaporated to the dryness to provide IVa.
1 H NMR (CDCl₃) δ = 1.34 (m, 1 H), 2.44 (m, 1 H), 3.02 (m, 1 H), 3.60-3.87 (m, 8H), 4.0 (m, 1 H), 4.56 (s, 2H), 7.27-7.37 (m, 10H) ppm.
13C NMR (CDCl₃) δ = 34.6, 52.3, 61.3, 69.3, 69.8, 73.3, 75.5, 76.2, 83.8, 127.1, 127.8, 128.2, 128.5, 128.6, 137.9, 140.0 ppm.

### Example 8: Preparation of (1S,2S,3R,5S)-3-Amino-5-(2-hydroxyethoxy)cyclopentane-1,2-diol (VI)

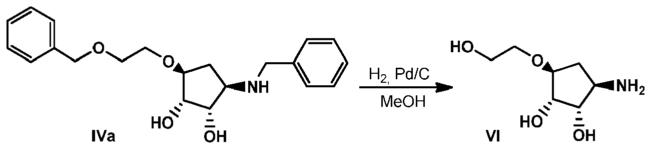

The solution of IVa (1.1 g, 2.8 mmol) in MeOH (15 mL) was hydrogenated at 10 bar of hydrogen for 16 hours in the presence of Pd/C (10 %, 0.2 g). The reaction mixture was passed through the pad of celite and evaporated to the dryness to provide VI.
1 H NMR (DMSO-d₆) δ = 1.07 (m, 1 H), 2.19 (m, 1 H), 2.90 (m, 1 H), 3.36-3.60 (m, 6H), 3.74 (m, 1 H) ppm.
13C NMR (DMSO-d₆) δ = 36.4, 55.0, 60.4, 70.7, 75.1, 78.7, 83.4 ppm.

### Example 9: Preparation of (3aS,4R,6S,6aR)-6-(2-(tert-butoxy)ethoxy)-2,2-dimethyltetrahydro-3aH-cyclopenta[d][1,3]dioxol-4-amine (Va)

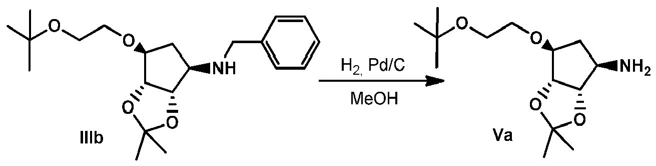

The solution of IIIb (6.0 g, 16.5 mmol) in MeOH (50 mL) was hydrogenated at 5 bar of hydrogen for 16 hours at 50 °C in the presence of Pd/C (10 %, 0.6 g). The reaction mixture was passed through the pad of celite and evaporated to the dryness to provide Va.
1 H NMR (CDCl₃) δ = 1.13 (s, 9H), 1.23 (s, 3H), 1.36 (s, 3H), 1.74 (d, 1 H), 2.06 (m, 1 H), 3.25 (d, 1 H), 3.43 (m, 2H), 3.55 (m, 2H), 3.81 (d, 1 H), 4.38 (d, 1 H), 4.61 (d, 1 H) ppm. 13C NMR (CDCl₃) δ = 23.8, 26.2, 27.4, 35.2, 57.8, 60.8, 68.8, 72.8, 84.0, 85.1, 88.5, 109.8 ppm.

### Example 10: Preparation of (1S,2S,3S,5R)-3-(2-(benzyloxy)ethoxy)-5-(tritylamino)cyclopentane-1,2-diol (IVb)

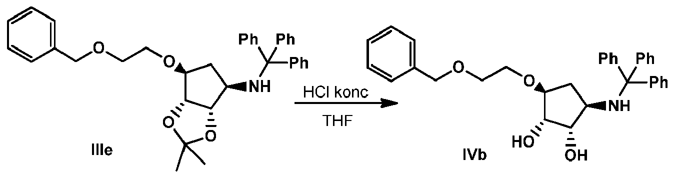

IIIe (1.0 g, 1.8 mmol) was treated with konc. HCl (0.5 mL) in THF (5 mL) at room temperature for 7 hours followed by addition of 8M NaOH (pH = 8). The reaction mixture was extracted with 20 mL of EtOAc. Organic phase was washed with water, dried over MgSO₄ and evaporated to the dryness to provide IVb.

1 H NMR (CDCl₃) δ = 0.85 (m, 1 H), 1.62 (m, 1h), 2.8 (s, 1 H), 2.94 (m, 1 H), 3.47 (m, 1 H), 3.56 (m, 4H), 3.77 (s, 1 H9, 3.90 (m, 1 H), 4.53 (s, 1 H), 7.20 (m, 3H) 7.28 (m, 11 H), 7.56 (d, 6H) ppm. 13C NMR (CDCl₃) δ = 36.2, 57.5, 60.4, 69.0, 70.0, 73.2, 75.2, 78.6, 83.4, 126.4, 127.7, 127.73, 127.9, 128.4, 128.8, 146.7 ppm.

### Example 11: Preparation of (1S,2S,3R,5S)-3-Amino-5-(2-hydroxyethoxy)cyclopentane-1,2-diol (VI)

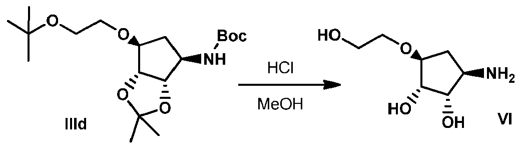

IIId (4.4 g, 11.8 mmol) was treated with conc. HCl (5 mL) in MeOH (20 mL) at reflux for 16 hours. MeOH was removed by evaporation and 10 mL of water was added to the residue. The mixture was washed with EtOAc (20 mL) followed by addition of 2M NaOH (pH = 9). Water was removed by evaporation and 30 mL of i-PrOH was added to the residue. After filtration, i-PrOH was removed under reduce pressure to provide VI.
1 H NMR (D₂O) δ = 1.52 (m, 1 H), 2.58 (m, 1 H), 3.33 (m, 1 H), 3.55-3.62 (m, 4 H), 3.79 (m, 1 H), 3.99 (m, 1H), 4.09 (m, 1 H) ppm.
13C NMR (D₂O) δ = 31.5, 53.9, 60.6, 70.5, 73.9, 74.2, 81.9 ppm.

### Example 12: Preparation of (1S,2S,3R,5S)-3-Amino-5-(2-hydroxyethoxy)cyclopentane-1,2-diol (VI)

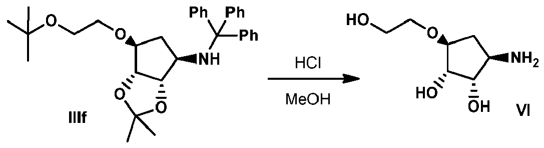

IIIf (1.0 g, 1.9 mmol) was treated with conc. HCl (2 mL) in MeOH (5 mL) at reflux for 16 hours. MeOH was removed by evaporation and 10 mL of water was added to the residue. The mixture was washed with EtOAc (10 mL) followed by addition of 2M NaOH (pH = 9). Water was removed by evaporation and 10 mL of i-PrOH was added to the residue. After filtration, i-PrOH was removed under reduce pressure to provide VI.
1 H NMR (D₂O) δ = 1.35 (m, 1 H), 2.51 (m, 1 H), 3.22 (m, 1 H), 3.60-3.65 (m, 4 H), 3.79 (m, 1 H), 3.85 (m, 1 H), 3.99 (m, 1 H) ppm.
13C NMR (D₂O) δ = 33.5, 53.9, 60.6, 70.5, 74.5, 76.1, 82.5 ppm.

### Example 13: Preparation of (1S,2S,3R,5S)-3-((5-amino-6-chloro-2-(propylthio)pyrimidin-4-yl)-amino)-5-(2-hydroxyethoxy)cyclopentane-1,2-diol (OLACINA)

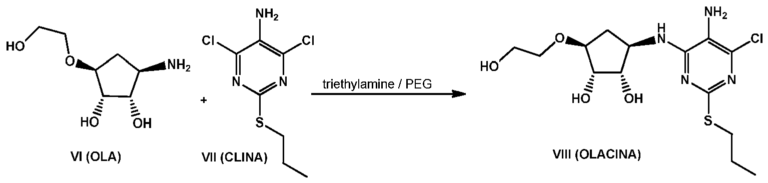

A mixture of (1*S*,2*S*,3*R*,5*S*)-3-amino-5-(2-hydroxyethoxy)cyclopentane-1,2-diol (**OLA;** 1.06 g, 6 mmol), 4,6-dichloro-2-(propylthio)pyrimidin-5-amine (**CLINA;** 1.43 g, 6 mmol), triethylamine (1.09 g, 7.8 mmol) and polyethyleneglycol PEG400 (2 mL) was stirred for 48 h at 75 °C. The reaction mixture was diluted with ethyl acetate (50 mL), washed with water (25 mL) and evaporated under reduced pressure to give a resinous material which solidified upon trituration in *n*-hexane (25 mL). After filtration there was obtained **OLACINA** as a grey powder (2.0 g, 88% yield): ¹³C NMR (DMSO-*d*₆, 125 MHz) δ 13.3, 22.8, 32.2, 34.7, 54.8, 60.4, 69.8, 72.4, 75.2, 82.4, 119.9, 137.5, 152.5, 155.1.

### Example 14: Preparation of (1S,2S,3R,5S)-3-(7-chloro-5-(propylthio)-3H-[1,2,3]triazolo[4,5-d]pyrimidin-3-yl)-5-(2-hydroxyethoxy)cyclopentane-1,2-diol (CLTOL)

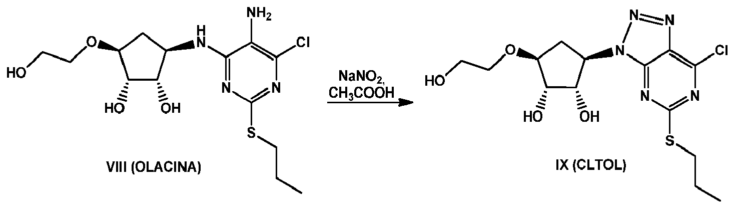

To a stirring solution of (1*S*,2*S*,3*R*,5*S*)-3-((5-amino-6-chloro-2-(propylthio)pyrimidin-4-yl)amino)-5-(2-hydroxyethoxy)cyclopentane-1,2-diol (**OLACINA;** 1.14 g, 3 mmol) in acetic acid (5 mL) was added sodium nitrite (0.23 g, 3.3 mmol) while maintaining the reaction temperature at 20-30 °C. The reaction mixture was stirred for 30 minutes. It was then diluted with ethyl acetate (50 mL), washed with water (2 x 35 mL) and evaporated under reduced pressure. The crude product was triturated in hexane (20 mL) and filtered to give **CLTOL** as an off-white powder (1.00 g, 86% yield): 98 area% by HPLC; mp 98-107 °C; ¹³C NMR (CDCl₃, 125 MHz) δ 13.4, 22.0, 33.5, 33.7, 61.75, 61.77, 71.1, 74.9, 75.4, 82.0, 131.8, 150.7, 152.9, 171.8.

### Example 15: Preparation of (1S,2S,3R,5S)-3-(7-chloro-5-(propylthio)-3H-[1,2,3]triazolo[4,5-d]pyrimidin-3-yl)-5-(2-hydroxyethoxy)cyclopentane-1,2-diol (CLTOL)

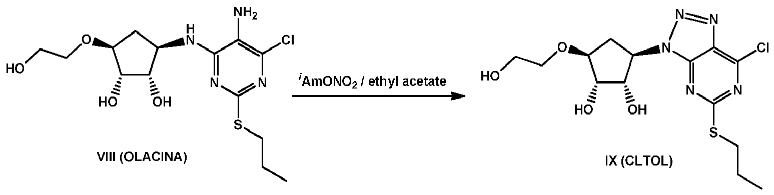

To a stirring solution of (1*S*,2*S*,3*R*,5*S*)-3-((5-amino-6-chloro-2-(propylthio)pyrimidin-4-yl)amino)-5-(2-hydroxyethoxy)cyclopentane-1,2-diol (**OLACINA**; 0.38 g, 1 mmol) in ethyl acetate (7 mL) was added isopentyl nitrite (0.16 mL, 1.2 mmol). The reaction mixture was stirred for 1 h at 65 °C and then evaporated under reduced pressure to give a reddish powder which was recrystallized from a mixture of methyl *tert*-butyl ether / *n*-hexane to give **CLTOL** as a crystalline product (0.31 g, 80% yield): 99 area% by HPLC.

### Example 16: Preparation of (1S,2S,3R,5S)-3-(7-chloro-5-(propylthio)-3H-[1,2,3]triazolo[4,5-d]pyrimidin-3-yl)-5-(2-hydroxyethoxy)cyclopentane-1,2-diol (CLTOL)

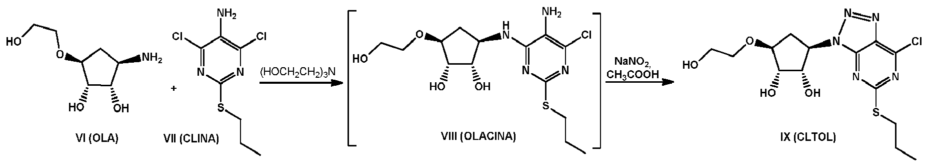

A mixture of (1S,2S,3R,5S)-3-amino-5-(2-hydroxyethoxy)cyclopentane-1,2-diol (OLA; 0.89 g, 5 mmol), 4,6-dichloro-2-(propylthio)pyrimidin-5-amine (CLINA; 1.19 g, 5 mmol) and triethanolamine (1.49 g, 10 mmol) was stirred neat for 24 h at 90 °C. After cooling to ambient temperature, the viscous reaction mixture was dissolved by the addition of acetic acid (3 mL). While maintaining the reaction temperature at 15-20 °C there was added sodium nitrite (0.38 g, 5.5 mmol) and the reaction mixture left stirring for 12 h. The reaction mixture was diluted with ethyl acetate (35 mL), washed with water (2 x 25 mL) and evaporated under reduced pressure. The crude product was triturated in hexane (20 mL) and filtered to give CLTOL as an off-white powder (1.61 g, 83% yield): ¹³C NMR (CDCl₃, 125 MHz) δ 13.4, 22.0, 33.5, 33.7, 61.75, 61.77, 71.1, 74.9, 75.4, 82.0, 131.8, 150.7, 152.9, 171.8.

### Example 17: Preparation of (1S,2S,3R,5S)-3-((6-chloro-5-nitro-2-(propylthio)pyrimidin-4-yl)amino)-5-(2-hydroxyethoxy)cyclopentane-1,2-diol (OLACIN)

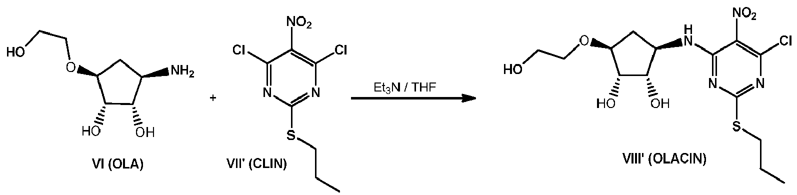

To a stirring solution of 4,6-dichloro-5-nitro-2-(propylthio)pyrimidine (**CLIN**; 0.80 g, 3 mmol) in tetrahydrofuran (30 mL) at 0 °C was slowly added a solution of (1S,2S,3R,5S)-3-amino-5-(2-hydroxyethoxy)cyclopentane-1,2-diol (**OLA**; 0.53 g, 3 mmol) in N-methylpyrrolidin-2-one (5 mL). After 10 min there was added triethylamine (0.42 mL, 3 mmol) and the mixture left stirring for 3 h at 0 °C and then 16 h at 20 °C. The reaction mixture was dilluted with ethyl acetate (60 mL), washed with water (2 × 60 mL) and evaporated under reduced pressure. The obtained crude product was triturated under diisopropyl ether (10 mL) and filtered to give **OLACIN** as a yellow powder (0.86 g, 70% yield): 98 area% by HPLC; mp 94-98 °C; ¹³C NMR (CDCl₃, 125 MHz) δ 13.2, 22.3, 32.7, 33.6, 55.7, 60.3, 70.7, 74.0, 74.6, 82.5, 124.5, 151.6, 153.9, 171.9.

### Example 18: Preparation of (1S,2S,3R,5S)-3-(7-chloro-5-(propylthio)-3H-[1,2,3]triazolo[4,5-d]pyrimidin-3-yl)-5-(2-hydroxyethoxy)cyclopentane-1,2-diol (CLTOL)

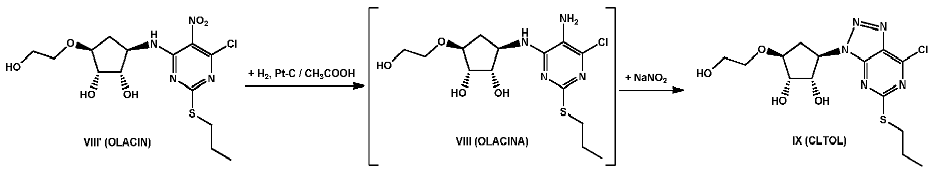

The solution of (1*S*,2*S*,3*R*,5*S*)-3-((6-chloro-5-nitro-2-(propylthio)pyrimidin-4-yl)amino)-5-(2-hydroxyethoxy)cyclopentane-1,2-diol (**OLACIN**; 0.74 g, 1.8 mmol) in acetic acid (15 mL) was hydrogenated for 5 h in the presence of platinum on carbon (5%; 0.11 g, 1.5 mol%) at 10 bar hydrogen pressure. The catalyst was filtered trough celite and washed with ethyl acetate (5 mL). The analysis of the filtrate confirmed complete conversion of the starting material and 95 area% HPLC purity of the intermediate (1*S*,2*S*,3*R*,5S)-3-((5-amino-6-chloro-2-(propylthio)pyrimidin-4-yl)amino)-5-(2-hydroxyethoxy)cyclopentane-1,2-diol (**OLACINA**). To the filtrate was then added sodium nitrite (0.14 g, 2 mmol) while stirring in a bath at 15-20 °C. After 1 h, the reaction mixture was diluted with ethyl acetate (50 mL), washed with water (2 x 50 mL) and evaporated under reduced pressure. The solid residue was triturated in n-hexane (10 mL) and filtered to give **CLTOL** as a grey powder (0.50 g, 71% yield): 96 area% by HPLC; mp 91-95 °C.

### Example 19: Preparation of ticagrelor (TCG)

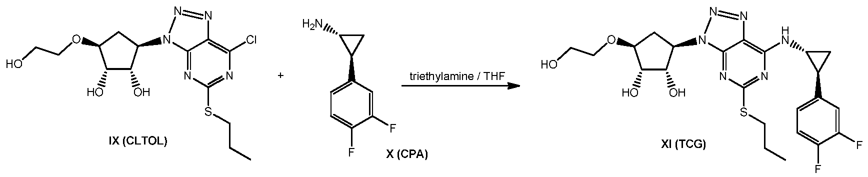

To a stirring solution of (1*S*,2*S*,3*R*,5*S*)-3-(7-(((1R,2S)-2-(3,4-difluorophenyl)cyclopropyl)amino)-5-(propylthio)-3*H*-[1,2,3]triazolo[4,5-d]pyrimidin-3-yl)-5-(2-hydroxyethoxy)cyclopentane-1,2-diol (**CLTOL**; 1.56 g, 4 mmol) in tetrahydrofuran (20 mL) at 20 °C was added a mixture of (1*R*,2*S*)-2-(3,4-difluorophenyl)cyclopropanamine (**CPA**; 0.68 g, 4 mmol) and triethylamine (0.70 mL, 5 mmol). After 2 h, the mixture was diluted with methyl *tert*-butyl ether (30 mL) and washed with 1% aqueous acetic acid (100 mL), water (75 mL) and evaporated under reduced pressure. The residue was triturated under *n*-hexane (20 mL) and filtered to give ticagrelor (2.00 g, 96% yield): ¹⁹F NMR (CDCl₃, 470.5 MHz) δ-142.4 (m, 1F), -139.0 (m, 1F); MS (ESI) *m*/*z*: 523 [MH]⁺.

### Example 20: Preparation of ticagrelor (TCG)

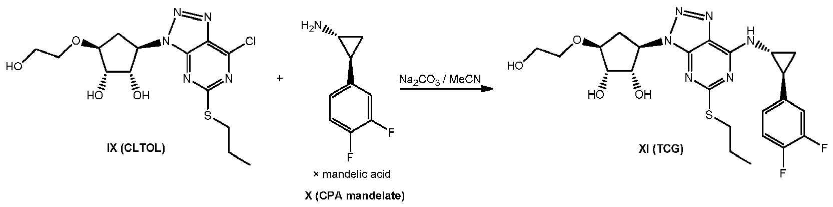

To a stirring suspension of (1*R*,2*S*)-2-(3,4-difluorophenyl)cyclopropanamonium mandelate (0.33 g, 1.03 mmol) and sodium carbonate (0.27 g, 2.5 mmol) in acetonitrile (12 mL) at 20 °C was added (1*S*,2*S*,3*R*,5*S*)-3-(7-(((1*R*,2*S*)-2-(3,4-difluorophenyl)cyclopropyl)amino)-5-(propylthio)-3*H-*[1,2,3]triazolo[4,5-d]pyrimidin-3-yl)-5-(2-hydroxyethoxy)cyclopentane-1,2-diol (**CLTOL**; 0.39 g, 1 mmol). The mixture was stirred for 20 h, diluted with water (50 mL) and extracted with ethyl acetate (60 mL). The extract was washed with water (50 mL), 0.1M aqueous acetic acid (50 mL), again water (50 mL) and evaporated under reduced pressure to give ticagrelor (0.50 g, 93% yield): ¹⁹F NMR (CDCl₃, 470.5 MHz) δ -142.5 (m, 1 F), -139.0 (m, 1 F); MS (ESI) *m*/*z*: 523 [MH]⁺.

## Claims

1. A process for the preparation of a compound of formula XI or a salt thereof 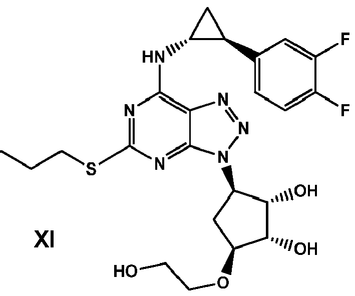 comprising the steps of:
(i) contacting a compound of formula VI or a salt thereof 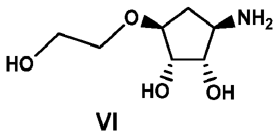 with a compound of formula VII or VII' 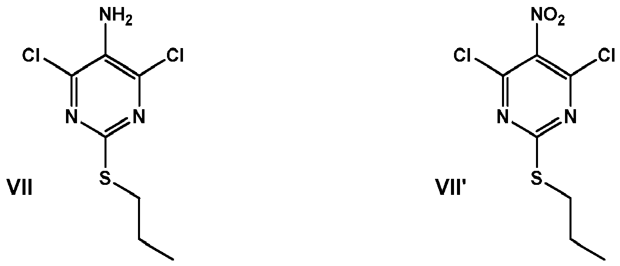 to obtain a compound of formula VIII or VIII', respectively 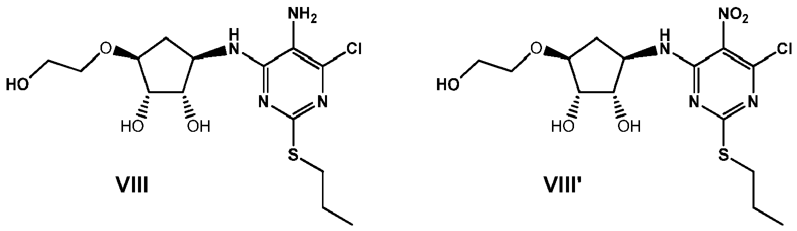
(ii) optionally, if a compound of formula VIII' is obtained, reducing the compound of formula VIII' to a compound of formula VIII,
(iii) converting a compound of formula VIII by nitrosation to a compound of formula IX 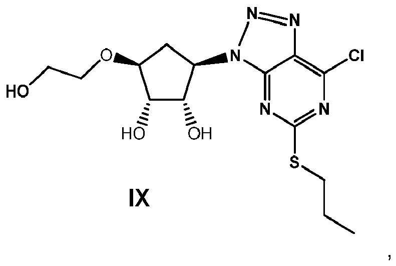 and
(iv) coupling the compound of formula IX with a compound of formula X 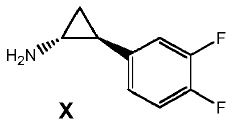 or a salt thereof in a presence of a base to provide a compound of formula XI or a salt thereof.

2. The process according to claim 1, wherein step (i), optional step (ii) and step (iii) are carried out in one pot.

3. The process according to claim 1, wherein the compound of formula VI or a salt thereof is prepared by comprising the steps of
either
(0-1) providing a compound of formula III or a salt thereof 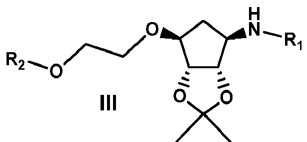 wherein R₁ is Bn, Boc, Cbz, TFA, Tr, TCA, CHO, Ac, Bz, t-Bu or Fmoc, and R₂ is Bn, t-Bu, TBDMS, MOM, Ac or Bz;
(0-2) directly converting a compound of formula III to a compound of formula VI, and
(0-3) optionally converting a compound of formula IV to a salt thereof; or
(0-1') providing a compound of formula I or a salt thereof 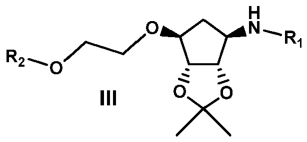 wherein R₁ and R₂ is as defined above,
(0-2') converting a compound of formula III to a compound of formula IV 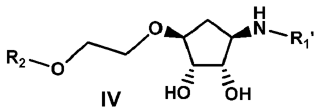 wherein R₁' is hydrogen, Bn, Boc, Cbz, TFA, Tr, TCA, CHO, Ac, Bz, t-Bu or Fmoc, and R₂ is as defined above, and
(0-3') converting a compound of formula IV to a compound of formula VI,
(0-4') optionally converting a compound of formula IV to a salt thereof; or
(0-1") providing a compound of formula III or a salt thereof 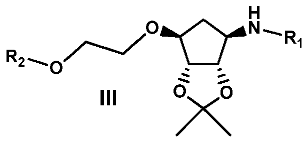 wherein R₁ and R₂ is as defined above,
(0-2") converting a compound of formula III to a compound of formula V 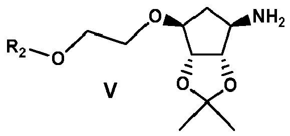 wherein R₂ is as defined above, and
(0-3") converting a compound of formula V to a compound of formula VI,
(0-4") optionally converting a compound of formula IV to a salt thereof.

4. The process according to claim 3, wherein the compound of formula III or a salt thereof is prepared by comprising the steps of:
(i) providing a compound of formula I 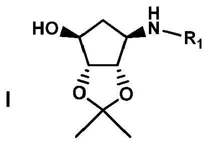 wherein R₁ is Bn, Boc, Cbz, TFA, Tr, TCA, CHO, Ac, Bz, *t*-Bu or Fmoc,
(ii) reacting the compound of formula I with a compound of formula II 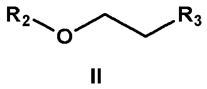 wherein R₂ is Bn, *t*-Bu, TBDMS, MOM, Ac or Bz, and R₃ is TsO, MsO, Br or Cl to yield a compound of formula III, and
(iii) optionally converting the compound of formula III to a salt thereof.

5. A process for the preparation of a pharmaceutical composition comprising a compound of formula XI or a pharmaceutically acceptable salt thereof 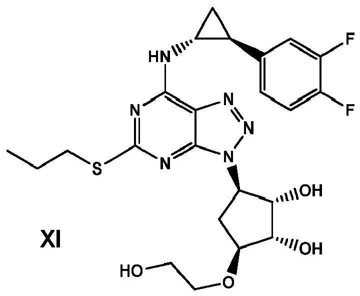 comprising the steps of:
(i) preparing a compound of formula XI or a salt thereof according to anyone of claims 1 to 4, optionally converting the salt into a pharmaceutically acceptable salt, and
(ii) mixing the compound of formula VIII or a pharmaceutically acceptable salt thereof with a pharmaceutically acceptable carrier and/or excipient.

6. A compound of the following formula III or a salt thereof 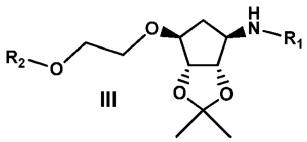 wherein R₁ is Bn, Boc, Cbz, TFA, Tr, TCA, CHO, Ac, Bz, t-Bu or Fmoc, and R₂ is Bn, *t*-Bu, TBDMS, MOM, Ac or Bz.

7. A compound selected from the fumarate salt, maleate salt or oxalate salt of the compound
of formula IIIa or IIIb

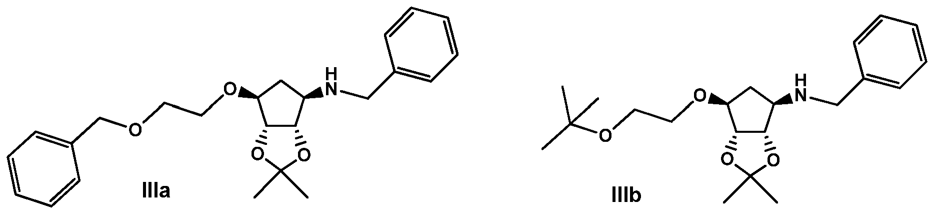

8. A compound of the following formula IV or a salt thereof 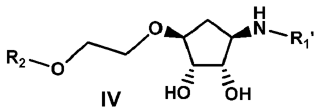 wherein R₁ is hydrogen, Bn, Boc, Cbz, TFA, Tr, TCA, CHO, Ac, Bz, *t*-Bu or Fmoc, and R₂ is Bn, t-Bu, TBDMS, MOM, Ac or Bz.

9. A compound of the following formula III or a salt thereof 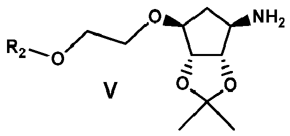 wherein R₂ is Bn, *t*-Bu, TBDMS, MOM, Ac or Bz.

10. A compound selected from the fumarate salt, maleate salt or oxalate salt of the compound of formula Va

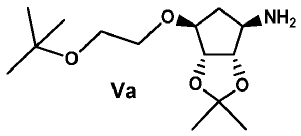

11. A compound of the following formula VIII or VIII'

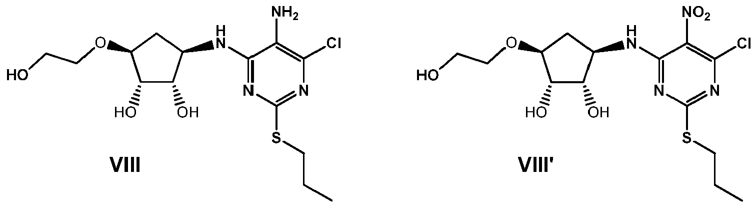

12. A compound of the following formula IX

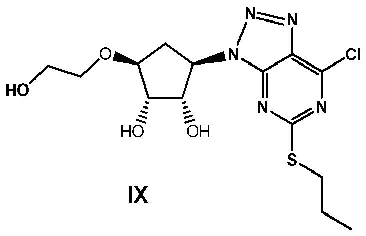

13. Use of a compound as defined in any one of claims 6 to 12 in the preparation of ticagrelor.
